# EUROPÄISCHE PATENTANMELDUNG

(11) **EP 3 944 866 A1**
(43) Veröffentlichungstag der Anmeldung: **02.02.2022**
(21) Anmeldenummer: 21186480.6
(22) Anmeldetag: 19.07.2021
(51) Int. Cl.: A61L 2/24, D06F 15/00, D06F 39/00, D06F 95/00, D06F 35/00, A61L 2/18

(54) **VERFAHREN UND VORRICHTUNG ZUM WASCHEN EINER MATRATZE ODER EINES MATRATZENKERNS**

(30) Priorität: 27.07.2020 DE 102020119760
(71) Anmelder: Rosenbaum, Jens, 31275 Lehrte (DE)
(72) Erfinder: Rosenbaum, Jens, 31275 Lehrte (DE)
(74) Vertreter: Holz, Christian

(57) **Zusammenfassung**

Die vorliegende Erfindung betrifft ein Verfahren zum Waschen wenigstens einer Matratze (3) oder wenigstens eines Matratzenkerns (3) mit wenigstens den Schritten:
• erstes Stauchen (100a; 200b) der Matratze (3) bzw. des Matratzenkerns (3) zumindest im Wesentlichen senkrecht zu ihrer bzw. zu seiner flächigen Erstreckungsrichtung innerhalb oder außerhalb einer Reinigungsflüssigkeit (2),
• erstes Entstauchen (300) der Matratze (3) bzw. des Matratzenkerns (3) innerhalb der Reinigungsflüssigkeit (2) und
• zweites Stauchen (400a; 500b) der Matratze (3) bzw. des Matratzenkerns (3) zumindest im Wesentlichen senkrecht zu ihrer bzw. zu seiner flächigen Erstreckungsrichtung innerhalb oder außerhalb der Reinigungsflüssigkeit (2).

Die vorliegende Erfindung betrifft ferner eine Matratzenwaschvorrichtung (1), vorzugsweise zur Durchführung eines Verfahrens wie zuvor beschrieben, mit wenigstens einer Wanneneinheit (10), welche ausgebildet ist, eine Reinigungsflüssigkeit (2) in einem Innenraum (10a) aufzunehmen, und mit wenigstens einer Hubeinheit (13), welche ausgebildet ist, wenigstens eine Matratze (3) oder wenigstens einen Matratzenkern (3) aufzunehmen, wobei die Hubeinheit (13) ferner ausgebildet ist, die aufgenommene Matratze (3) bzw. den auf-genommenen Matratzenkern (3), vorzugsweise zumindest im Wesentlichen senkrecht zu ihrer bzw. zu seiner flächigen Erstreckungsrichtung, besonderes vorzugsweise in der vertikalen Richtung (Z), vorzugsweise gegenüber einem Gestell (11), in den Innenraum (10a) der Wanneneinheit (10) hinein und/oder aus dem Innenraum (10a) der Wanneneinheit (10) heraus zu bewegen, wobei die Hubeinheit (13) ferner ausgebildet ist, die aufgenommene Matratze (3) bzw. den auf-genommenen Matratzenkern (3) zumindest im Wesentlichen senkrecht zu ihrer bzw. zu seiner flächigen Erstreckungsrichtung, vorzugsweise in der vertikalen Richtung (Z), zu stauchen und zu entstauchen.

## Beschreibung

Die vorliegende Erfindung betrifft ein Verfahren zum Waschen wenigstens einer Matratze oder wenigstens eines Matratzenkerns gemäß dem Patentanspruch 1 sowie eine Matratzenwaschvorrichtung gemäß des Patentanspruchs 12.

Um den Menschen das Liegen, insbesondere zum Schlafen, angenehmer und insbesondere erholsamer zu gestalten, sind seit Langem Matratzen bekannt. Matratzen werden heutzutage hierzu üblicherweise auf einen Lattenrost, auf eine Unterfederung oder auf ein ähnliches Polster gelegt. Diese Kombination wird üblicherweise als Bettsystem und in Kombination mit einem Rahmen oder mit einem Gestell als Bett bezeichnet.

Eine Matratze weist üblicherweise einen Kern, auch Matratzenkern genannt, zum Beispiel aus Schaumstoff, aus Latex, aus Naturprodukten oder aus einem Federkern auf, welcher von einem Bezug, auch Matratzenbezug genannt, aus einem üblicherweise weichen aber festen textilem Gewebe umschlossen wird. Neben häufig verwendeten Materialien wie Baumwolle und Viskose werden heute vor allem moderne Synthetikfasern wie Polyamid, Polyester, Mikrofaser oder Lyocell für den Matratzenbezug verwendet, die einlagig, aber auch als dreidimensionale Abstandsgewirke gefertigt werden. Manche Hersteller verwenden auch Leinen, Seide und Wolle.

Aufgrund der heutzutage abnehmbaren Matratzenbezüge ist es bekannt, zur Reinigung einer Matratze dessen Matratzenbezug vom Matratzenkern zu entfernen und separat bzw. einzeln zum Beispiel in einer Waschmaschine zu waschen oder anderweitig zu reinigen. Soll die Matratze lediglich oberflächig gereinigt werden, so kann der Matratzenbezug auch auf dem Matratzenkern verbleiben und der Matratzenbezug kann von außen zum Beispiel abgesaugt oder mit einem feuchten Tuch oder dergleichen lokal behandelt werden.

Um den Matratzenkern zu reinigen, können ebenfalls Staubsauger oder vergleichbare Geräte verwendet werden, um lose Fremdkörper von der Oberfläche des Matratzenkerns zu entfernen, wobei die Saugkraft des Staubsaugers in Verbindung mit dem Aufbau des Matratzenkerns entscheidet, ob auch tieferliegende Fremdkörper erreicht und aus dem Matratzenkern entfernt werden können. Ein derartiger Staubsauger kann an seinem Saugkopf auch rotierende Bürsten aufweisen, um Verschmutzungen von der Oberfläche des Matratzenkerns zu lösen und dann abzusaugen. Auf diese Art und Weise kann auch der Matratzenbezug auf dem Matratzenkern gereinigt werden.

Um die Wirkung und insbesondere die Tiefenwirkung in das Material des Matratzenkerns hinein gegenüber dem rein oberflächigen Absaugen zu erhöhen, sind verschiedene Ergänzungen zum Absaugen bekannt, welche auch miteinander kombiniert eingesetzt werden können. Diese Ergänzungen zum Absaugen können auch auf die Matratze als Ganzes, d.h. mit Matratzenbezug über dem Matratzenkern, angewendet werden.

Beispielsweise ist seitens der Firma POTEMA GmbH eine Matratzen-Reinigungsmaschine bekannt, welche neben dem Absaugen auch hochfrequente Schwingungen erzeugen kann, so dass Schmutzpartikel wie zum Beispiel Milbenkot als Auslöser von Hausstauballergien im Inneren der Matratze bzw. des Matratzenkerns gelöst, pulverisiert und in einem genau auf Matratzen abgestimmten Vakuum entfernt werden kann. Gleichzeitig kann eine intensive UV-Strahlung eine weitgehende Minimierung von Bakterien, Viren und Sporen bewirken.

Auch sind beispielsweise seitens der Firma Alfred Kärcher Vertriebs-GmbH Waschsauger u.a. für Matratzen bekannt, welche eine Flüssigkeit als Reinigungslösung mit Druck in die Fasern der Matratze bzw. des Matratzenkerns einsprühen und die Reinigungslösung zusammen mit dem gelösten Schmutz wieder absaugen können.

Vorteilhaft ist bei derartigen Geräten für die Reinigung von Matratzen bzw. von Matratzenkernen, dass diese Geräte klein, kompakt, beweglich, kostengünstig und bzw. oder von einer Person transportierbar und bzw. oder einsetzbar sein können. Insbesondere lose Oberflächenverschmutzen von Matratzen bzw. von Matratzenkernen lassen sich hierdurch schnell, einfach, kostengünstig und bzw. oder flexibel entfernen.

Nachteilig ist jedoch, dass eine wesentliche Verschmutzung von Matratzen bzw. von Matratzenkernen hierdurch gar nicht oder nur unzureichend bzw. nur oberflächig entfernt werden kann, nämlich der natürliche menschliche Schweiß, welcher Salze und Eiweiße enthält. Derartige Verschmutzungen können auch andere Körperflüssigkeiten sein, die während einer Nacht ausgeschieden werden können. Diese Salze und Eiweiße können durch den Schweiß in flüssiger oder in dampfförmiger Form auch tief in den Matratzenkern eindringen und nach dem Trocknen dort verbleiben. Mittels der zuvor beschriebenen Reinigungsgeräte bzw. -verfahren können derartige tief in die Matratze bzw. in den Matratzenkern eingedrungene Verschmutzungen und insbesondere dort am bzw. im Material des Matratzenkerns angetrocknete Verschmutzungen jedoch gar nicht entfernt werden.

So reicht die Saugwirkung von Staubsaugern kaum in die Matratze bzw. in den Matratzenkern hinein und kann ferner gar keine getrockneten Verschmutzungen lösen und hierdurch absaugbar machen.

Hieran ändert auch die Verwendung von rotierenden Bürsten nichts, da diese lediglich an der Oberfläche wirken können. Durch die Bürsten kann es vielmehr zu zusätzlichen Abnutzungen bzw. zu Beschädigungen der Oberfläche der Matratze bzw. des Matratzenkerns kommen.

Auch durch den Einsatz von hochfrequenten Schwingungen können angetrocknete Verschmutzungen nicht gelöst werden. Ferner kann die Saugwirkung derartiger Matratzen-Reinigungsmaschinen nicht in die Tiefe der Matratze bzw. des Matratzenkerns dringen und Verschmutzungen von dort absaugen.

Ebenso ist die Wirkung von UV-Strahlung in die Matratze bzw. in den Matratzenkern hinein begrenzt und kann diese bzw. diesen nicht vollständig durchdringen bzw. in der Tiefe erreichen. Auch dient der Einsatz der UV-Strahlung dem Entkeimen bzw. dem Desinfizieren des Materials; angetrocknete Verschmutzungen können durch UV-Strahlung jedoch gar nicht gelöst oder entfernt werden.

Um angetrocknete Verschmutzungen aus dem Material einer Matratze bzw. eines Matratzenkerns zu lösen und zu entfernen kann daher Dampf eingesetzt werden, welcher von einem Reinigungshandgerät erzeugt und über eine Düse lokal auf die Oberfläche der Matratze bzw. des Matratzenkerns gerichtet werden kann.

Nachteilig ist hierbei jedoch, dass der Dampf, welcher lediglich eine Temperatur von gut 100°C aufweist, oberflächennah in der Matratze bzw. in dem Matratzenkern durch den Kontakt mit dem zu durchdringenden Material abkühlt, kondensiert und als Flüssigkeit kaum tiefergehend in das Material des Matratzenkerns eindringen kann. Daher bleibt die lösende bzw. reinigende Wirkung von Dampf hinsichtlich angetrockneter Verschmutzungen auch auf den oberflächennahen Bereich des Matratzenkerns beschränkt. Dies gilt ebenso für die absaugende Wirkung derartiger Handgeräte.

Nachteilig ist bei der Verwendung von Flüssigkeit bzw. Dampf zur Reinigung von Matratzen bzw. von Matratzenkernen generell, dass der Flüssigkeit bzw. dem Dampf zugesetzte Chemikalien als Reinigungsmittel in dem Material des Matratzenkerns zurückbleiben und selbst eine angetrocknete Verschmutzung darstellen können. Auch kann Flüssigkeit in dem Matratzenkern verbleiben und zum Beispiel zu Schimmel führen. Um dies zu vermeiden kann eine zusätzliche Trocknung der mit Flüssigkeit bzw. mit Dampf behandelten Matratzenkerne erforderlich sein, was jedoch einen zusätzlichen Aufwand darstellen sowie zusätzliche Zeit erfordern kann.

Nachteilig ist bei der Verwendung von Dampf, welcher wenigstens eine Temperatur von 100°C aufweisen muss, um dampfförmig zu sein, sowie von heißen Flüssigkeiten, dass deren hohe Temperaturen zu Beschädigungen des Materials der Matratze bzw. des Matratzenkerns führen können.

Belastungen bzw. Beschädigungen des Materials der Matratze bzw. des Matratzenbezugs können auch durch das Saugen von Luft und ggfs. Flüssigkeit auftreten, da durch das Ansaugen die Kett- und Schußfäden eines Gewebes des Matratzenbezugs angehoben und gedehnt werden können, anschließend jedoch die Fasern der Kett- und Schußfäden ggfs. nicht mehr an ihre ursprüngliche Position zurückkehren.

Nachteilig beim Einsatz von Flüssigkeiten ist ferner, dass ein Absaugen der Flüssigkeit eine möglichst glatte Oberfläche der Matratze bzw. des Matratzenkerns erfordert, um die erforderliche Saugwirkung zu erzielen. Daher können Waschsauger lediglich bei glatten bzw. bei ebenen Oberflächen wirkungsvoll eingesetzt werden.

Seitens der Firma Hartmann GmbH ist die Dampfsterilisationsanlage "VS 36 L" bekannt, welche der Desinfektion von Bettausstattungen dient. Entsprechend ist die Dampfsterilisationsanlage "VS 36 L" als Standgerät mit einer Kammer ausgelegt, welche bis zu 12 Matratzen gleichzeitig aufnehmen kann. Die Kammer kann mittels einer Tür dampfdicht verschlossen und der Innenraum der Kammer mit Dampf gefüllt werden.

Nachteilig bleibt hierbei, dass weiterhin lediglich die Oberflächen der Matratzen bzw. der Matratzenkerne von dem Dampf erreicht und dort oberflächennah desinfiziert werden können. Insbesondere können ggfs. lediglich die seitlichen Kanten der Matratzen bzw. der Matratzenkerne vom Dampf erreicht werden, wenn mehrere Matratzen bzw. Matratzenkerne senkrecht zu ihrer flächigen Erstreckungsrichtung aufeinandergestapelt werden und hierdurch einander flächig abdecken.

Nachteilig ist insbesondere, dass der verflüssigte Dampf in den Matratzen bzw. in den Matratzenkernen verbleibt bzw. im Gegensatz zu entsprechenden Reinigungshandgeräten nicht aus den Matratzen bzw. aus den Matratzenkernen abgesaugt wird. Entsprechend können selbst dann, wenn angetrocknete Verschmutzungen im Matratzenkern von verflüssigtem Dampf erreicht und gelöst wurden, diese nicht bzw. nur unzureichend durch selbsttätigen Abfließen aus dem Matratzenkern entfernt werden.

Dies kann die reinigende Wirkung von Dampfsterilisationsanlagen gegenüber Reinigungshandgeräten sogar verschlechtern, weshalb derartige Dampfsterilisationsanlagen üblicherweise lediglich in Krankenhäusern bei speziellen Matratzen angewendet werden, welche einen luft-, feuchtigkeits- und dampfundurchlässigen Bezug aufweisen, sodass dort gar keine Verschmutzungen in die Matratze bzw. in den Matratzenkern eindringen können und daher eine reine Oberflächenbehandlung mittels Dampf vollkommen ausreichend ist. Derartige spezielle Matratzen werden aufgrund der unkomfortablen Oberflächeneigenschaften daher lediglich in Krankenhäusern und dergleichen eingesetzt, jedoch nicht zum Beispiels im heimischen Bereich oder in Hotels, deren Gästen ein möglichst komfortables Liegen bzw. Schlafen auf "normalen" Matratzen geboten werden soll.

Ferner sind Waschmaschinen für Matratzen bekannt, welche mit dem Wirkprinzip von Trommelwaschmaschinen funktionieren, wie sie üblicherweise in Haushalten verwendet werden. Die Waschtrommel, die in einem schließbaren Innenraum der Matratzentrommelwaschmaschine rotieren kann, ist dabei in mehrere Kammern unterteilt, welche jeweils eine Matratze aufnehmen können. Derartige Matratzentrommelwaschmaschinen sind beispielsweise von der Firma A3S bekannt, siehe zum Beispiel WO 2011 086 428 A1.

Bei einer derartigen Matratzentrommelwaschmaschine befindet sich die flüssige Waschmittellösung als Reinigungsflüssigkeit im unteren Teil des geschlossenen Innenraums. Durch Rotation der Waschtrommel wird jede ihrer Kammern und somit jede Matratze durch die Waschmittellösung getaucht, so dass die Matratzen von der Waschmittellösung erreicht und gereinigt werden können. Dies kann auch für angetrocknete Verschmutzungen im Matratzenkern gelten. Das Entfernen der Waschmittellösung mit den gelösten Fremdstoffen erfolgt durch die Zentrifugalkräfte bei beschleunigtem Drehen der Waschtrommel, nachdem die Waschmittellösung aus dem Innenraum abgepumpt wurde.

Nachteilig hierbei ist jedoch, dass die Waschmittellösung von alleine in das Material der Matratze bzw. dessen Kern eindringen muss. Die Tiefenwirkung der Waschmittellösung ist dabei abhängig von der Diffusionsfähigkeit des Matratzenmaterials bzw. des Materials des Matratzenkerns, womit bestimmte Teilbereiche des Matratzenmaterials gegebenenfalls unerreicht bleiben, was die Tiefenwirkung erheblich einschränkt.

Das Wirkprinzip einer Waschtrommel im Haushalt setzt zudem nicht nur auf das Tauchen des zu waschenden Materials in der Waschtrommel sowie deren Rotation, sondern auch darauf, dass die textilen Materialen in der Trommel gegenseitig physisch aufeinander einwirken, so dass hierdurch Verschmutzungen an den Fasern bzw. im Material mechanisch bearbeitet und gelöst werden können. Bei einer Matratzentrommelwaschmaschine jedoch werden die Matratzen, damit sie nicht durch die Zentrifugalkräfte bzw. Fliehkräfte zerrissen werden, ganz bewusst in Kammern fixiert, wodurch die notwendige, zusätzliche mechanische Bearbeitung entfällt. Dies kann die Waschwirkung der Matratzen bzw. der Matratzenkerne in einer Matratzentrommelwaschmaschine gegenüber dem Wirkprinzip heimischer Trommelwaschmaschinen mindern.

Nachteilig ist auch, dass die Fliehkräfte zum Entfernen der Waschmittellösung aus den Matratzen bzw. aus der Matratzenkernen eine hohe Belastung für das Material der Matratzen bzw. der Matratzenkerne darstellen können. Dies kann die Lebensdauer der Matratzen bzw. der Matratzenkerne reduzieren.

Nachteilig ist ferner der enorme Aufwand, den eine Matratzentrommelwaschmaschine hinsichtlich ihrer Größe, ihres Gewichts, ihres Wasserverbrauchs, ihres Energieverbrauchs und bzw. oder ihrer Anschaffungs- und Betriebskosten verursachen kann. Insbesondere kann das Antreiben der Waschtrommel aus dem Stand auf die Betriebsdrehzahl einen hohen Energieeinsatz erfordern, was zu entsprechend hohen Betriebskosten führen kann.

Nachteilig ist des Weiteren, dass das Beladen der Matratzentrommelwaschmaschine mit zu waschenden Matratzen bzw. mit zu waschenden Matratzenkernen und bzw. oder das Entladen gewaschener Matratzen bzw. gewaschener Matratzenkerne für die entsprechende Person umständlich und bzw. oder anstrengend sein kann.

Nachteilig ist auch, dass die Matratzen bzw. die Matratzenkerne, insbesondere bei Schaummatratzen, nach dem Waschen und Schleudern dennoch eine vergleichsweise hohe Restfeuchte aufweisen können. Diese kann ein zusätzliches Trocknen in einem entsprechenden Matratzentrockner erfordern, was zu weiteren Kosten, Platzbedarf, Gewicht, Energieverbrauch und bzw. oder Anschaffungs- und Betriebskosten führen kann. Auch ist eine zusätzliche Bedienung bzw. Befüllung und Entleerung des Matratzentrockners durch eine Person erforderlich. In jedem Fall kostet der zusätzliche Trocknungsvorgang, insbesondere ohne Matratzentrockner, Zeit, in welcher die Matratzen bzw. die Matratzenkerne nicht verwendet werden können.

Eine Aufgabe der vorliegenden Erfindung ist es, eine Matratzenwaschvorrichtung der eingangs beschriebenen Art bereit zu stellen, so dass Verschmutzungen wirkungsvoller, materialschonender, kostengünstiger, platzsparender, gewichtssparender, energiesparsamer und bzw. oder schneller als bisher bekannt aus einer Matratze und bzw. oder aus einem Matratzenkern gelöst und entfernt werden können. Dies soll insbesondere in der gesamten Tiefe der Matratze bzw. des Matratzenkerns erfolgen können. Dies soll insbesondere für angetrocknete Verschmutzungen erreicht werden können. Zusätzlich oder alternativ soll die Restfeuchte der Matratze bzw. des Matratzenkerns gegenüber bekannten Matratzenwaschvorrichtungen reduziert werden können. Zumindest soll eine Alternative zu bekannten Verfahren und bzw. oder zu bekannten Vorrichtungen zum Waschen von Matratzen und bzw. oder von Matratzenkernen geschaffen werden.

Die Aufgabe wird erfindungsgemäß durch ein Verfahren mit den Merkmalen des Patentanspruchs 1 sowie durch eine Matratzenwaschvorrichtung mit den Merkmalen des Patentanspruchs 12 gelöst. Vorteilhafte Weiterbildungen sind in den Unteransprüchen beschrieben.

Somit betrifft die vorliegende Erfindung ein Verfahren zum Waschen wenigstens einer Matratze, d. h. eines Matratzenkerns mit Matratzenbezug, oder wenigstens eines Matratzenkerns mit wenigstens den Schritten:
- erstes Stauchen der Matratze bzw. des Matratzenkerns zumindest im Wesentlichen senkrecht zu ihrer bzw. zu seiner flächigen Erstreckungsrichtung innerhalb oder außerhalb einer Reinigungsflüssigkeit,
- erstes Entstauchen der Matratze bzw. des Matratzenkerns innerhalb der Reinigungsflüssigkeit und
- zweites Stauchen der Matratze bzw. des Matratzenkerns zumindest im Wesentlichen senkrecht zu ihrer bzw. zu seiner flächigen Erstreckungsrichtung innerhalb oder außerhalb der Reinigungsflüssigkeit.

Unter einem Stauchen wird ein Zusammendrücken, ein Zusammenpressen bzw. ein Komprimieren des Materials der Matratze bzw. des Matratzenkerns verstanden. Entsprechend wird unter einem Entstauchen das Aufheben des zusammengedrückten, des zusammengepressten bzw. des komprimierten Zustands des Materials der Matratze bzw. des Matratzenkerns verstanden. Dies erfolgt jeweils zumindest im Wesentlichen in der Richtung, welche senkrecht zur flächigen Erstreckung der Matratze bzw. des Matratzenkerns ausgerichtet ist. Mit anderen Worten erfolgt das Stauchen zumindest im Wesentlichen in der Richtung, in welcher die Matratze bzw. der Matratzenkern die kürzeste Erstreckung aufweist, d. h. am dünnsten ist. Als Reinigungsflüssigkeit kann insbesondere Wasser verwendet werden, welches vorzugsweise mit einem Reinigungsmittel versetzt sein kann, um die reinigende Wirkung der Reinigungsflüssigkeit zu erhöhen.

Mit anderen Worten wird das erfindungsgemäße Verfahren derart ausgeführt, dass das Material der Matratze als Ganzes oder lediglich des Matratzenkerns nach Entfernen des Matratzenbezugs, welcher separat wie herkömmlich bekannt gewaschen werden kann, durch das erste Stauchen derart zusammengedrückt wird, dass die Luft zumindest teilweise bzw. abschnittsweise und vorzugsweise möglichst vollständig aus dem Material der Matratze bzw. des Matratzenkerns entfernt wird. Dies kann in Kontakt mit der Reinigungsflüssigkeit bzw. vollständig eingetaucht innerhalb der Reinigungsflüssigkeit ebenso erfolgen wie außerhalb der Reinigungsflüssigkeit zum Beispiel an der Umgebungsluft.

Entscheidend ist, dass das anschließende Entstauchen der Matratze bzw. des Matratzenkerns erst innerhalb der Reinigungsflüssigkeit erfolgt, worunter sowohl ein Kontakt mit der Reinigungsflüssigkeit als auch ein vollständiges Eintauchen in die Reinigungsflüssigkeit fallen. Durch das Entstauchen kann sich somit das Material der Matratze bzw. des Matratzenkerns zumindest abschnittsweise und vorzugsweise vollständig mit der Reinigungsflüssigkeit vollsaugen, sodass die Reinigungsflüssigkeit möglichst tiefgehend bis vollständig tiefgehend in das Material der Matratze bzw. des Matratzenkerns eindringen kann. Hierdurch können insbesondere angetrocknete Verschmutzungen auch tief im Material der Matratze bzw. des Matratzenkerns von der Reinigungsflüssigkeit erreicht und gelöst werden.

Durch das anschließende zweite Stauchen kann dann die Reinigungsflüssigkeit samt der aufgenommenen Verschmutzungen wieder möglichst vollständig aus dem Material der Matratze bzw. des Matratzenkerns herausgepresst werden, sodass hierdurch die von der Reinigungsflüssigkeit gelösten Verschmutzungen aus dem Material der Matratze bzw. des Matratzenkerns heraustransportiert und entfernt werden können. Dies kann sowohl in Kontakt mit der Reinigungsflüssigkeit, vollständig in der Reinigungsflüssigkeit eingetaucht sowie außerhalb der Reinigungsflüssigkeit erfolgen.

Auf diese Art und Weise kann erfindungsgemäß eine Matratze bzw. ein Matratzenkern tiefgehend mit Reinigungsflüssigkeit gewaschen und insbesondere im Material der Matratze bzw. des Matratzenkerns angetrocknete Verschmutzungen wie zum Beispiel Bestandteile des menschlichen Schweißes gelöst und aus der Matratze bzw. dem Matratzenkern nach außen hin abgeführt werden. Insbesondere tief im Material der Matratze bzw. des Matratzenkerns befindliche angetrocknete Verschmutzungen können durch das Einsaugen der Reinigungsflüssigkeit beim Entstauchen erreicht und durch das anschließende zweite Stauchen aus dem Material der Matratze bzw. des Matratzenkerns nach außen hin herausgepresst werden. Dies kann zu einer hohen Reinigungswirkung führen.

Vorteilhaft ist hierbei auch, dass im Vergleich zu Matratzentrommelwaschmaschinen die Belastungen des Materials der Matratze bzw. des Matratzenkerns vermieden werden können, welche dort durch die Fliehkräfte beim Schleudern auftreten können.

Vorteilhaft ist hierbei ferner, dass durch das zweite Stauchen außerhalb der Reinigungsflüssigkeit die vom Material der Matratze bzw. des Matratzenkerns aufgesogen Reinigungsflüssigkeit wieder weitestgehend aus dem Material der Matratze bzw. des Matratzenkerns herausgedrückt werden kann, sodass insbesondere bei Schaummatratzen die verbleibende Restfeuchtigkeit im Material der Matratze bzw. des Matratzenkerns geringgehalten werden kann.

Gemäß einem Aspekt der Erfindung weist das Verfahren wenigstens die weiteren Schritte auf:
- erstes Stauchen der Matratze bzw. des Matratzenkerns zumindest im Wesentlichen senkrecht zu ihrer bzw. zu seiner flächigen Erstreckungsrichtung außerhalb der Reinigungsflüssigkeit und
- Eintauchen der Matratze bzw. des Matratzenkerns in die Reinigungsflüssigkeit.

In diesem Fall erfolgt das erste Stauchen der Matratze bzw. des Matratzenkerns außerhalb der Reinigungsflüssigkeit und damit bevor die Matratze bzw. der Matratzenkern in die Reinigungsflüssigkeit eingetaucht wird. Mit anderen Worten wird die Matratze bzw. der Matratzenkern erstmalig im trockenen Zustand außerhalb des Reservoirs der Reinigungsflüssigkeit, d. h. in der Umgebungsluft, gestaucht, was das erste Stauchen einfacher und bzw. oder mit weniger Kraftaufwand durchführbar machen kann. Auf diese Art und Weise kann die Matratze bzw. der Matratzenkern im gestauchten Zustand in die Reinigungsflüssigkeit eingetaucht werden, sodass dort das Entstauchen als nächster Schritt ausgeführt werden kann.

Dabei können das erste Stauchen der Matratze bzw. des Matratzenkerns und dessen Eintauchen in die Reinigungsflüssigkeit als voneinander getrennte Schritte zeitlich nacheinander erfolgen oder die Bewegungen dieser beiden Schritte können miteinander kombiniert ausgeführt werden, um die Durchführung des erfindungsgemäßen Verfahrens zu verkürzen.

Gemäß einem weiteren Aspekt der Erfindung weist das Verfahren wenigstens die weiteren Schritte auf:
- Eintauchen der Matratze bzw. des Matratzenkerns in die Reinigungsflüssigkeit und
- erstes Stauchen der Matratze bzw. des Matratzenkerns zumindest im Wesentlichen senkrecht zu ihrer bzw. zu seiner flächigen Erstreckungsrichtung innerhalb der Reinigungsflüssigkeit.

Mit anderen Worten wird die Reihenfolge der Schritte des ersten Stauchen und des Eintauchens gegenüber dem zuvor beschriebenen Aspekte der Erfindung nun umgedreht, sodass sich die Matratze bzw. der Matratzenkern bereits in der Reinigungsflüssigkeit eingetaucht befindet, wenn das Material der Matratze bzw. des Matratzenkerns zum ersten Mal gestaucht wird. Vorteilhaft ist hierbei, dass das Material der Matratze bzw. des Matratzenkerns innerhalb der Reinigungsflüssigkeit auch ohne Stauchen die Reinigungsflüssigkeit zumindest teilweise aufsaugen kann, sodass sich bereits hierdurch Verschmutzungen lösen können. Erfolgt nun das erste Stauchen der Matratze bzw. des Matratzenkerns innerhalb der Reinigungsflüssigkeit, so können zumindest diese gelösten Verschmutzungen bereits durch das erste Stauchen aus dem Material der Matratze bzw. des Matratzenkerns entfernt werden. Dies kann die reinigende Wirkung des erfindungsgemäßen Verfahrens zumindest für Randbereiche des Materials der Matratze bzw. des Matratzenkerns erhöhen.

Gemäß einem weiteren Aspekt der Erfindung weist das Verfahren wenigstens die weiteren Schritte auf:
- zweites Stauchen der Matratze bzw. des Matratzenkerns zumindest im Wesentlichen senkrecht zu ihrer bzw. zu seiner flächigen Erstreckungsrichtung innerhalb der Reinigungsflüssigkeit und
- Herausbewegen der Matratze bzw. des Matratzenkerns aus der Reinigungsflüssigkeit.

Auf diese Art und Weise kann das möglichst vollständige Entfernen der Reinigungsflüssigkeit aus dem Material der Matratze bzw. des Matratzenkerns erfolgen, während sich die Matratze bzw. der Matratzenkern noch innerhalb der Reinigungsflüssigkeit befindet. Hierdurch kann die Reinigungsflüssigkeit, welche durch das zweite Stauchen aus dem Material der Matratze bzw. des Matratzenkerns herausgepresst wird, direkt in die übrige Reinigungsflüssigkeit übergehen, wodurch ein Austreten der herausgepressten Reinigungsflüssigkeit an die Umgebung verhindert werden kann.

Auch kann die gestauchte Matratze bzw. der gestauchte Matratzenkern ein deutlich geringeres Gewicht aufweisen als die mit Reinigungsflüssigkeit vollgesogene Matratze bzw. als der mit Reinigungsflüssigkeit vollgesogene Matratzenkern, sodass die gestauchte Matratze bzw. der gestauchte Matratzenkern mit einem geringeren Kraftaufwand aus dem Reservoir der Reinigungsflüssigkeit herausbewegt werden kann als eine ungestauchte vollgesogene Matratze bzw. ein ungestauchter vollgesogener Matratzenkern. Dies kann die Verwendung von Antrieben mit geringerer Leistung und damit mit geringerem Gewicht, mit geringeren Bauraum und bzw. oder mit geringeren Kosten zur Durchführung des Herausbewegens der Matratze bzw. des Matratzenkerns ermöglichen. Hierdurch kann auch der Platzbedarf bzw. die Stellfläche für eine entsprechende Matratzenwaschvorrichtung reduziert werden. Auch kann hierdurch der Energieverbrauch reduziert werden, welcher für das Herausbewegen erforderlich sein kann. Dies kann sich entsprechend reduzierend auf die Erwärmung dieser Antriebe auswirken.

Das zweite Stauchen der Matratze bzw. des Matratzenkerns und dessen Herausbewegen aus der Reinigungsflüssigkeit können ebenfalls als voneinander getrennte Schritte zeitlich nacheinander erfolgen oder die Bewegungen dieser beiden Schritte können miteinander kombiniert ausgeführt werden, um die Durchführung des erfindungsgemäßen Verfahrens zu verkürzen.

Gemäß einem weiteren Aspekt der Erfindung weist das Verfahren wenigstens die weiteren Schritte auf:
- Herausbewegen der Matratze bzw. des Matratzenkerns aus der Reinigungsflüssigkeit und
- zweites Stauchen der Matratze bzw. des Matratzenkerns zumindest im Wesentlichen senkrecht zu ihrer bzw. zu seiner flächigen Erstreckungsrichtung außerhalb der Reinigungsflüssigkeit.

Mit anderen Worten wird die Reihenfolge der Schritte des zweiten Stauchens und des Herausbewegens gegenüber dem zuvor beschriebenen Aspekte der Erfindung nun umgedreht, sodass das zweite Stauchen der Matratze bzw. des Matratzenkerns erst erfolgt, nachdem die Matratze bzw. der Matratzenkern aus der Reinigungsflüssigkeit herausbewegt wurde. In diesem Fall können die Reinigungsflüssigkeit aus dem Material der Matratze bzw. des Matratzenkerns durch das zweite Stauchen herausgepresst werden, wenn sich die Matratze bzw. der Matratzenkern außerhalb des Reservoirs der Reinigungsflüssigkeit befindet.

Gemäß einem weiteren Aspekt der Erfindung weist das Verfahren wenigstens den weiteren Schritt auf:
- zweites Entstauchen der Matratze bzw. des Matratzenkerns außerhalb der Reinigungsflüssigkeit.

Mit anderen Worten wird durch das zweite Entstauchen der Matratze bzw. des Matratzenkerns das vorangehende zweite Stauchen wieder aufgehoben, sodass die Matratze bzw. der Matratzenkern freigegeben und zum Beispiel einer entsprechenden Hubeinheit, wie weiter unten noch näher beschrieben werden wird, zum Beispiel durch eine Automatisierungsvorrichtung oder von einer Person entnommen werden kann.

Gemäß einem weiteren Aspekt der Erfindung weist das Verfahren wenigstens die weiteren Schritte auf:
- außerhalb der Reinigungsflüssigkeit, Aufnehmen der Matratze bzw. des Matratzenkerns, vorzugsweise in einer Hubeinheit, und bzw. oder
- außerhalb der Reinigungsflüssigkeit, Entnehmen der Matratze bzw. des Matratzenkerns, vorzugsweise aus der Hubeinheit.

Mit anderen Worten kann die zu waschende Matratze bzw. der zu waschende Matratzenkern zum Beispiel in einer entsprechenden Hubeinheit zum Beispiel von einer Automatisierungsvorrichtung oder von einer Person angeordnet werden, sodass diese Hubeinheit die zuvor beschriebenen Verfahrensschritte zumindest teilweise ausführen kann. Aus dieser Hubeinheit kann die gewaschene Matratze bzw. der gewaschene Matratzenkern abschließend zum Beispiel von einer Automatisierungsvorrichtung oder von einer Person wieder entnommen werden. Dies kann die automatische bzw. selbsttätige Durchführung der zuvor beschriebenen Verfahrensschritte seitens der Hubeinheit ermöglichen, welche ein Bestandteil einer Matratzenwaschvorrichtung sein kann, wie weiter unten noch näher beschrieben werden wird.

Gemäß einem weiteren Aspekt der Erfindung werden die Schritte des Stauchens und des Entstauchens mehrfach innerhalb der Reinigungsflüssigkeit ausgeführt. Hierdurch kann die reinigende Wirkung des abwechselnden Stauchens und Entstauchens innerhalb der Reinigungsflüssigkeit und des hierdurch bewirkten Einsaugens und Herauspressens der Reinigungsflüssigkeit in bzw. aus dem Material der Matratze bzw. des Matratzenkerns erhöht werden, indem diese Schritte wiederholt ausgeführt werden.

Gemäß einem weiteren Aspekt der Erfindung werden die Schritte des zweiten Stauchens und des zweiten Entstauchens außerhalb der Reinigungsflüssigkeit mehrfach ausgeführt. Hierdurch kann die trocknende Wirkung des abwechselnden Stauchens und Entstauchens außerhalb der Reinigungsflüssigkeit und des hierdurch bewirkten Herauspressens der verbleibenden Reinigungsflüssigkeit aus dem Material der Matratze bzw. des Matratzenkerns erhöht werden, indem diese Schritte wiederholt ausgeführt werden.

Gemäß einem weiteren Aspekt der Erfindung erfolgt das Eintauchen in die Reinigungsflüssigkeit zumindest im Wesentlichen senkrecht zur flächigen Erstreckungsrichtung der Matratze bzw. des Matratzenkerns, vorzugsweise in der vertikalen Richtung, und bzw. oder erfolgt das Herausbewegen aus der Reinigungsflüssigkeit zumindest im Wesentlichen senkrecht zur flächigen Erstreckungsrichtung der Matratze bzw. des Matratzenkerns, vorzugsweise in der vertikalen Richtung, erfolgt. Dies kann die Ausbildung einer entsprechenden Matratzenwaschvorrichtung zumindest in der Höhe vergleichsweise kompakt halten. Auch kann dies die Wegstrecke vergleichsweise gering halten, über welche insbesondere die Bewegungen des Eintauchens und bzw. oder des Herausbewegens ausgeführt werden.

Gemäß einem weiteren Aspekt der Erfindung ist die Reinigungsflüssigkeit aufgeheizt. Dies kann die reinigende Wirkung der Reinigungsflüssigkeit erhöhen bzw. erhalten.

Die vorliegende Erfindung betrifft auch eine Matratzenwaschvorrichtung, vorzugsweise zur Durchführung eines Verfahrens wie zuvor beschrieben, mit wenigstens einer Wanneneinheit, welche ausgebildet ist, eine Reinigungsflüssigkeit in einem Innenraum aufzunehmen. Die Wanneneinheit kann auch als Reservoir der Reinigungsflüssigkeit bezeichnet werden. Die Wanneneinheit kann vorzugsweise von mehreren Seitenwänden und einem Boden gebildet werden, sodass ein quaderförmiger Innenraum der Wanneneinheit gebildet wird, welcher die Reinigungsflüssigkeit aufnehmen und in der vertikalen Richtung von oben zugänglich sein kann. Auf diese Art und Weise kann eine zu waschende Matratze bzw. ein zu waschender Matratzenkern zumindest im Wesentlichen in der vertikalen Richtung von oben in den Innenraum der Wanneneinheit hineinbewegt sowie aus diesem herausbewegt werden.

Die Matratzenwaschvorrichtung weist ferner wenigstens eine Hubeinheit auf, welche ausgebildet ist, wenigstens eine Matratze oder wenigstens einen Matratzenkern aufzunehmen, wobei die Hubeinheit ferner ausgebildet ist, die aufgenommene Matratze bzw. den aufgenommenen Matratzenkern, vorzugsweise zumindest im Wesentlichen senkrecht zu ihrer bzw. zu seiner flächigen Erstreckungsrichtung, besonderes vorzugsweise in der vertikalen Richtung, vorzugsweise gegenüber einem Gestell, in den Innenraum der Wanneneinheit hinein und bzw. oder aus dem Innenraum der Wanneneinheit heraus zu bewegen, wobei die Hubeinheit ferner ausgebildet ist, die aufgenommene Matratze bzw. den aufgenommenen Matratzenkern zumindest im Wesentlichen senkrecht zu ihrer bzw. zu seiner flächigen Erstreckungsrichtung, vorzugsweise in der vertikalen Richtung, zu stauchen und zu entstauchen. Dies kann vorzugsweise die Umsetzung des erfindungsgemäßen Verfahrens mit seinen verschiedenen Aspekten wie zuvor beschrieben aber auch alternative Verfahren ermöglichen.

Mit anderen Worten kann die Hubeinheit eine Struktur darstellen, welche gegenüber einer übergeordneten Struktur wie zum Beispiel einem Gestell der Matratzenwaschvorrichtung relativ bewegt werden kann. Die übergeordnete Struktur wie zum Beispiel das Gestell der Matratzenwaschvorrichtung kann dabei feststehend in der Umgebung der Wanneneinheit angeordnet und zum Beispiel auf einem Untergrund, an einer Wand und bzw. oder an einer Decke eines Raums eines Gebäudes befestigt sein, sodass die Hubeinheit Bewegungen gegenüber der Umgebung der Wanneneinheit ausführen kann. Die Hubeinheit kann somit in die Reinigungsflüssigkeit hinein bzw. aus dieser heraus verfahren werden. Zusätzlich kann die Hubeinheit die aufgenommene Matratze bzw. den aufgenommenen Matratzenkern durch Relativbewegungen innerhalb der Hubeinheit stauchen und entstauchen.

Alternativ kann die Hubeinheit bzw. dessen Struktur an sich selbst feststehend angeordnet sein und die Bewegungen der Hubeinheit in die Reinigungsflüssigkeit hinein bzw. aus der Reinigungsflüssigkeit heraus kann durch Relativbewegungen innerhalb der Hubeinheit, bzw. durch zwei Stauchelemente, wie weiter unten näher beschrieben werden wird, vorgenommen werden, indem diese entsprechend weit verfahren werden. Hierdurch können die Bewegungen in die Reinigungsflüssigkeit hinein und aus der Reinigungsflüssigkeit heraus von denselben Elementen innerhalb der Hubeinheit ausgeführt werden, welche auch das Stauchen und Entstauchen ausführen können.

Die Bewegungen der Hubeinheit in den Innenraum der Wanneneinheit hinein und bzw. oder aus dem Innenraum der Wanneneinheit heraus können dabei beliebig sein, um wenigstens eine von der Hubeinheit aufgenommene Matratze bzw. einen von der Hubeinheit aufgenommenen Matratzenkern in den Innenraum der Wanneneinheit hineinzubewegen und bzw. oder aus dem Innenraum der Wanneneinheit herauszubewegen. Dies kann vorzugsweise auf vergleichsweise kurzem Wege und bzw. oder schnell zumindest im Wesentlichen senkrecht und vorzugsweise genau senkrecht zur flächigen Erstreckungsrichtung der Matratze bzw. des Matratzenkerns erfolgen. Insbesondere können diese Bewegungen der Hubeinheit in der vertikalen Richtung erfolgen, was den Weg besonders kurz halten und bzw. oder besonders schnell erfolgen kann.

Die Bewegungen können mittels entsprechender Antriebe erfolgen, welche mittels einer Steuerungseinheit der Matratzenwaschvorrichtung gesteuert bzw. betrieben werden können. Seitens der Steuerungseinheit kann auch das entsprechende Verfahren zumindest im Wesentlichen selbsttätig ablaufen. Als Antriebe können zum Beispiel hydraulische Antriebe, pneumatische Antriebe oder elektrische Antriebe verwendet werden.

Vorteilhaft ist hierbei auch, dass aufgrund der zumindest im Wesentlichen und vorzugsweise vollständig rein translatorischen Bewegung der Hubeinheit gegenüber der Wanneneinheit die erfindungsgemäße Matratzenwaschvorrichtung vergleichsweise kompakt und insbesondere deutlich kleiner als bekannte Matratzentrommelwaschmaschinen ausgebildet werden kann. Dies kann es nicht nur ermöglichen, entsprechende Waschverfahren und insbesondere das zuvor beschriebene erfindungsgemäße Verfahren auf vergleichsweise kleinem Raum umzusetzen, sondern kann dies auch bei einem geringeren Gewicht der erfindungsgemäßen Matratzenwaschvorrichtung erfolgen. Dies gilt ebenso für einen reduzierten Aufwand an Ressourcen wie zum Beispiel Wasser, Reinigungsmittel und bzw. oder Energie. Auch können die Investitionskosten sowie die laufenden Kosten für Betrieb und Wartung einer erfindungsgemäßen Matratzenwaschvorrichtung deutlich geringer als bei bekannten Matratzentrommelwaschmaschinen ausfallen.

Vorteilhaft ist ferner, dass die Hubeinheit vergleichsweise einfach von einer Automatisierungsvorrichtung oder von einer Person mit wenigstens einer zu waschenden Matratze bzw. mit wenigstens einem zu waschenden Matratzenkern bestückt und bzw. oder eine gewaschene Matratze bzw. ein gewaschener Matratzenkern aus der Hubeinheit entnommen werden kann. Dies kann den entsprechenden Aufwand geringhalten und bzw. oder die Durchführung dieser Schritte mit geringem Zeitaufwand ermöglichen.

Gemäß einem Aspekt der Erfindung weist die Hubeinheit wenigstens ein erstes Stauchelement und ein zweites Stauchelement auf, welche ausgebildet sind, zwischen sich die Matratze bzw. den Matratzenkern, vorzugsweise zumindest im Wesentlichen senkrecht zu ihrer bzw. zu seiner flächigen Erstreckungsrichtung, besonderes vorzugsweise in der vertikalen Richtung, aufzunehmen, wobei wenigstens das erste Stauchelement ausgebildet ist, innerhalb der Hubeinheit relativ zum zweiten Stauchelement zumindest im Wesentlichen senkrecht zur flächigen Erstreckungsrichtung der aufgenommenen Matratze bzw. des aufgenommenen Matratzenkerns, vorzugsweise in der vertikalen Richtung, bewegt zu werden und hierdurch die aufgenommene Matratze bzw. den aufgenommenen Matratzenkern zumindest im Wesentlichen senkrecht zu ihrer bzw. zu seiner flächigen Erstreckungsrichtung, vorzugsweise in der vertikalen Richtung, zu stauchen und zu entstauchen.

Mit anderen Worten ist, wie zuvor beschrieben, die Hubeinheit zum einen als Ganzes ausgebildet, relativ zum Gestell der Matratzenwaschvorrichtung in den Innenraum der Wanneneinheit hinein und aus dem Innenraum der Wanneneinheit heraus bewegt zu werden. Zum anderen weist die Hubeinheit zwei Stauchelemente auf, welche ausgebildet sind, wenigstens eine Matratze bzw. wenigstens einen Matratzenkern flächig zwischen sich aufzunehmen und eine stauchende Wirkung von beiden Seiten der flächigen Erstreckung der Matratze bzw. des Matratzenkerns auf die Matratze bzw. auf den Matratzenkern auszuüben. Die beiden Stauchelemente können daher auch als Stauchböden bezeichnet werden. Die beiden Stauchelemente können insbesondere als Lochbleche ausgebildet sein, um die Reinigungsflüssigkeit durch ihre Löcher hindurch fließen lassen zu können.

Zum Ausüben der stauchenden Wirkung kann wenigstens das erste Stauchelement innerhalb der Hubeinheit mittels eines entsprechenden Antriebs zu dem zweiten Stauchelement hin bzw. von dem zweiten Stauchelement weg beweglich bzw. verfahrbar ausgebildet sein. Hierdurch kann das erste Stauchelement zum zweiten Stauchelement hin bewegt werden, um ein Stauchen durchzuführen, indem die zwischen den beiden Stauchelementen aufgenommene Matratze bzw. der zwischen den beiden Stauchelementen aufgenommene Matratzenkern von dem ersten Stauchelement gegen das zweite Stauchelement gepresst und hierdurch zusammengedrückt bzw. komprimiert wird. Dieser gestauchte Zustand der Matratze bzw. des Matratzenkerns kann durch ein Entstauchen wieder aufgehoben werden, indem das erste Stauchelement wieder vom zweiten Stauchelement weg bewegt wird.

Gemäß einem weiteren Aspekt der Erfindung ist das zweite Stauchelement feststehend an der Hubeinheit angeordnet oder das zweite Stauchelement ist ausgebildet, innerhalb der Hubeinheit relativ zum ersten Stauchelement zumindest im Wesentlichen senkrecht zur flächigen Erstreckungsrichtung der aufgenommenen Matratze bzw. des aufgenommenen Matratzenkerns, vorzugsweise in der vertikalen Richtung, bewegt zu werden und hierdurch die aufgenommene Matratze bzw. den aufgenommenen Matratzenkern zumindest im Wesentlichen senkrecht zu ihrer bzw. zu seiner flächigen Erstreckungsrichtung, vorzugsweise in der vertikalen Richtung, zu stauchen und zu entstauchen.

Somit kann das zweite Stauchelement zum einen als feststehender Bestandteil der Hubeinheit ausgebildet werden, gegenüber welcher das erste Stauchelement wie zuvor beschrieben verfahren werden kann. Dies kann die Umsetzung der Hubeinheit vereinfachen, da seitens des zweiten Stauchelements auf einen weiteren Antrieb verzichtet werden kann.

Zum anderen kann jedoch auch das zweite Stauchelement innerhalb der Hubeinheit zu dem ersten Stauchelement hin und von dem ersten Stauchelement weg verfahrbar ausgebildet werden. Hierdurch können die Bewegungen, welche wie zuvor beschrieben ein Stauchen und ein Entstauchen der zwischen den beiden Stauchelementen aufgenommenen Matratze bzw. des zwischen den beiden Stauchelementen aufgenommenen Matratzenkerns bewirken können, von beiden Stauchelementen gemeinsam ausgeführt werden. Die jeweiligen Bewegungen der beiden Stauchelemente können entsprechend kürzer ausfallen. Auch können die Vorgänge des Stauchens und des Entstauchens durch die Bewegungen der beiden Stauchelemente schneller durchgeführt werden, was die Durchführung des entsprechenden Verfahrens zum Waschen der Matratze bzw. des Matratzenkerns zeitlich verkürzen kann.

Gemäß einem weiteren Aspekt der Erfindung ist die Wanneneinheit, vorzugweise wenigstens eine Seitenwand und bzw. oder ein Boden der Wanneneinheit, zumindest abschnittsweise beheizbar ausgebildet. Hierdurch kann die Reinigungsflüssigkeit im Innenraum der Wanneneinheit erwärmt bzw. warmgehalten werden. Dies kann die reinigende Wirkung der Reinigungsflüssigkeit erhöhen bzw. erhalten.

Mit anderen Worten kann die erfindungsgemäße Matratzenwaschvorrichtung, welche auch als Matratzen-Stauch-Tauch-Waschmaschine bezeichnet werden kann, als ein Wannensystem mit Hubeinheit angesehen werden. Das Wirkprinzip der erfindungsgemäßen Matratzenwaschvorrichtung beruht auf dem Effekt, den offenporige sowie rückdeformationsfähige feste Stoffe wie zum Beispiel Schaummatratzen aufweisen, wenn sich unter bestimmten Umständen der sie umgebende Aggregatzustand von gasförmig in flüssig ändert. Ein offenporiger und rückdeformationsfähiger Stoff wie zum Beispiel ein Matratzenschaum enthält in seinen Poren nämlich zunächst die ihn umgebende Materie wie zum Beispiel die gasförmige Umgebungsluft. Beim Stauchen kann die Luft aus den Poren herausgedrückt werden. Entstaucht man das Material wieder, füllen sich die Poren des Materials der Matratze bzw. des Matratzenkerns bei der Rückdeformation wieder auf. Taucht man das Material der Matratze bzw. des Matratzenkerns vor dem Entstauchen in eine Flüssigkeit, so füllen sich die Poren dann entsprechend mit der Flüssigkeit auf, welche durch ein anschließendes Stauchen ebenfalls wieder aus den Poren des Materials herausgepresst werden kann. Das Stauchen und Entstauchen in und außerhalb einer Flüssigkeit kann somit zu einer Sog- und Auspresswirkung führen, mit welcher Reinigungsflüssigkeiten das Material einer Matratze bzw. eines Matratzenkerns möglichst vollständig durchdringen und die durch die Reinigungsflüssigkeit als Waschsubstanz gelösten Schmutzpartikeln möglichst vollständig entfernen können. Dafür eignen sich insbesondere Schaummatratzen, da diese einem Schwamm am ähnlichsten sind.

Eine zur Durchführung des erfindungsgemäßen Verfahrens geeignete erfindungsgemäße Matratzenwaschvorrichtung kann eine Wanne aufweisen, welche über ein kontrolliertes Zu- und Ablaufsystem von Reinigungsflüssigkeiten mit Wasser und Reinigungsmittel verfügen kann. Diese Zu- und Ablaufsysteme können mit entsprechenden Speichern bzw. Tanks verbunden sein, um einen wechselseitigen Austausch von Flüssigkeiten zu ermöglichen. Alternativ, im Falle eines Einsatzes bei professionellen Wäschereien, kann die Wanne an deren Rein- und Schmutzwassersystem angeschlossen werden. Im beziehungsweise über dem Boden der Wanne und bzw. oder in den Wänden der Wanne kann ein Heizsystem integriert werden, um die Reinigungsflüssigkeit zu erwärmen bzw. warm zu halten und hierdurch die Wirksamkeit der Waschsubstanzen zu gewährleisten, sollte kein ausreichend vortemperiertes Wasser bzw. Reinigungsflüssigkeit von außen zugeführt werden können.

Die Wanne bzw. die Wanneneinheit kann mit einer doppelten Hubeinheit mit zwei Lochblechen als Stauchelemente bzw. als flüssigkeitsdurchlässige Bleche ausgestattet sein. Dabei können die Stauchelemente sowohl einzeln als auch zusammen in derselben sowie in der entgegengesetzten Richtung angehoben und abgesenkt werden. Diese unterschiedlichen Bewegungen der beiden Stauchelemente können ein Eintauchen sowie ein Herausheben der Matratze bzw. des Matratzenkerns in und aus der Reinigungsflüssigkeit sowie das Stauchen und Entstauchen der Matratze bzw. des Matratzenkerns ermöglichen. Eine beispielhafte Bauausführung einer erfindungsgemäßen Matratzenwaschvorrichtung kann in säurebeständigem Nirosta-Edelstahl erfolgen und hinsichtlich ihrer Abmessungen Außenmaß von ca. 250 cm in der Länge, von ca. 150 cm in der Breite und von ca. 250 cm in der Höhe aufweisen.

Beispielsweise können mit dem Einsatz lediglich einer Wanneneinheit zwei bis drei Matratzen bzw. Matratzenkerne, je nach Höhe der Matratzen bzw. der Matratzenkerne, gleichzeitig gewaschen werden, wobei ein kompletter Waschzyklus ca. 20 min dauern kann. Das Be- und Entladen der Hubeinheit mit den Matratzen bzw. mit den Matratzenkernen kann manuell erfolgen, indem die Matratzen bzw. Matratzenkerne horizontal auf das untere Stauchelement gelegt werden.

Falls lediglich eine Wanneneinheit zur Verfügung steht, müssen für die Vorwäsche (bei Bedarf), für die Hauptwäsche und für die Spülung jeweils die entsprechenden Reinigungsflüssigkeiten zum Beispiel als Wasser mit oder ohne Reinigungsmittel zugeführt, abgepumpt und zwischengespült werden. Daher kann für einen wirtschaftlichen Praxisbetrieb ein Mehrwannensystem sinnvoll sein, bei welchem zwei bzw. drei erfindungsgemäße Matratzenwaschvorrichtungen für die Vorgänge Vorwaschen, Hauptwaschen und Spülen zur Verfügung stehen können. Dabei kann die Matratze bzw. der Matratzenkern zwischen den Hubeinheiten der einzelnen Wanneneinheiten zum Beispiel via Rollenlager wechseln.

Ein erfindungsgemäßes Verfahren kann mittels einer erfindungsgemäßen Matratzenwaschvorrichtung vorzugsweise teilweise oder vollständig wie folgt durchgeführt werden:
1. Die zu reinigende Matratze kann in ihre Komponenten Matratzenbezug und Matratzen(Schaum-)Kern zerlegt werden.
2. Der Matratzenbezug kann dann separat und auf herkömmliche Weise zum Beispiel in einer Trommelwaschmaschine gewaschen und anschließend getrocknet werden. Alternativ kann der Matratzenbezug auch auf dem Matratzenkern verbleiben und die Matratze als Ganzes erfindungsgemäß gewaschen werden.
3. Der Matratzenkern kann horizontal zwischen die beiden Lochbleche als Stauchelemente eingeführt werden und auf dem unteren Lochblech als zweites Stauchelement zu liegen kommen.
4. Der Innenraum der Wanneneinheit kann zuvor, parallel oder anschließend mit einer Reinigungslösung befüllt werden.
5. Die Hubeinheit kann nun die beiden Lochbleche in der vertikalen Richtung aufeinander zu bewegen und hierdurch den dazwischenliegenden Matratzenkern in der vertikalen Richtung stauchen.
6. Die Hubeinheit kann parallel oder anschließend die beiden Lochbleche in der vertikalen Richtung in die Reinigungsflüssigkeit im Innenraum der Wanneneinheit verfahren und hierdurch den zwischen den Lochblechen gestauchten Matratzenkern in die Reinigungsflüssigkeit drücken.
7. Die Hubeinheit kann die Lochbleche samt Matratzenkern vollständig in die Reinigungsflüssigkeit der Wanne eintauchen lassen, um dann die beiden Lochbleche voneinander weg zu bewegen und hierdurch den Matratzenkern in der Reinigungsflüssigkeit zu entstauchen. Der Matratzenkern kann sich dabei mit der Reinigungsflüssigkeit vollsaugen.
8. Die Hubeinheit kann die beiden Lochbleche wieder aufeinander zu verfahren und hierdurch den Matratzenkern erneut stauchen. Dadurch kann auf physikalisch-mechanische Weise die Reinigungsflüssigkeit samt gelösten Verunreinigungen aus dem Matratzenkern schonend herausgepresst werden. Dies gilt ebenso für das Hineinsaugen beim Entstauchen.
9. Der Vorgang des Stauchens und Entstauchens kann bei Bedarf innerhalb der Reinigungsflüssigkeit mehrfach wiederholt werden.
10. Die Hubeinheit kann nach Abschluß des Waschvorgangs den Matratzenkern mittels der beiden Lochbleche wieder aus der Reinigungslösung herausheben, wobei der Matratzenkern gestaucht bleiben kann. Auf diese Weise kann der Matratzenkern weniger Flüssigkeit beim Herausheben enthalten.
11. Es kann ein mehrfaches Stauchen und Entstauchen des Matratzenkerns außerhalb der Reinigungsflüssigkeit erfolgen, um Restflüssigkeit aus dem Matratzenkern zu entfernen.
12. Die verschmutzte Reinigungsflüssigkeit kann aus dem Innenraum der Wanneneinheit ausgeleitet werden.
13. Der Innenraum der Wanneneinheit kann mit klarem Wasser gespült werden.
14. Der Innenraum der Wanneneinheit kann mit klarem Wasser befüllt werden.
15. Die Hubeinheit kann den Matratzenkern mittels der Lochbleche in das klare Wasser im Innenraum der Wanneneinheit verfahren, um ein Spülen durchzuführen.
16. Durch erneutes Stauchen und Entstauchen des Matratzenkerns innerhalb des klaren Wassers können Rückstände der Reinigungsflüssigkeit entfernt werden.
17. Nach erfolgter Klarspülung kann der Matratzenkern über die Hubeinheit wieder aus dem Innenraum der Wanneneinheit gehoben werden.
18. Der Matratzenkern kann zum Entfernen von Wasser mehrfach außerhalb des Innenraums der Wanneneinheit gestaucht und entstaucht werden.
19. Der Matratzenkern kann aus der Hubeinheit entnommen und zur Trocknung der Restfeuchte in einen Trockenraum mit heißer Belüftung gegeben werden.

Mehrere Ausführungsbeispiele und weitere Vorteile der Erfindung werden nachstehend im Zusammenhang mit den folgenden Figuren rein schematisch dargestellt und näher erläutert. Darin zeigt:
- Fig. 1: eine perspektivische schematische Darstellung einer erfindungsgemäßen Matratzenwaschvorrichtung gemäß eines ersten Ausführungsbeispiels;
- Fig. 2 bis 5: verschiedene schematische seitliche Darstellungen der erfindungsgemäßen Matratzenwaschvorrichtung gemäß des ersten Ausführungsbeispiels;
- Fig. 6: eine schematische seitliche Darstellung einer erfindungsgemäßen Matratzenwaschvorrichtung gemäß eines zweiten Ausführungsbeispiels;
- Fig. 7 und 8: schematische seitliche Darstellungen einer erfindungsgemäßen Matratzenwaschvorrichtung gemäß eines dritten Ausführungsbeispiels;
- Fig. 9 bis 18: verschiedene Schritte eines erfindungsgemäßen Verfahrens anhand der erfindungsgemäßen Matratzenwaschvorrichtung gemäß des ersten Ausführungsbeispiels; und

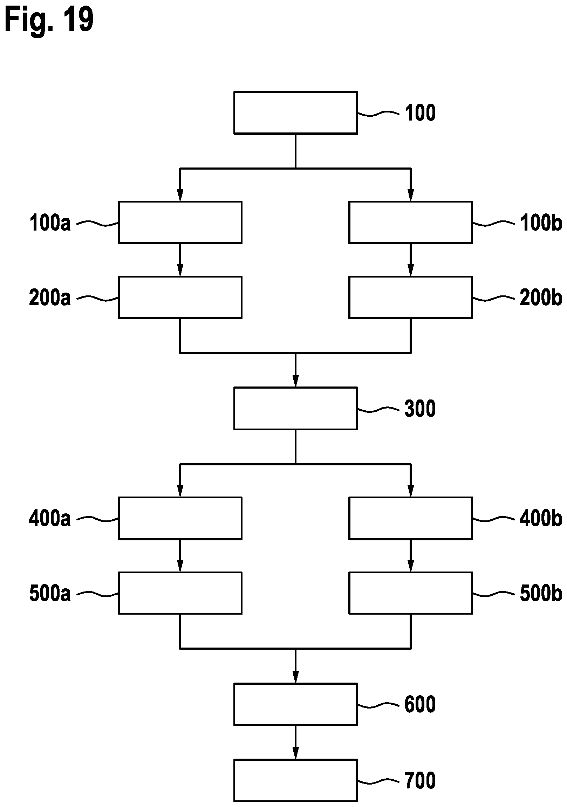

- Fig. 19: ein Ablaufdiagramm des erfindungsgemäßen Verfahrens.

Die o.g. Figuren werden in kartesischen Koordinaten betrachtet. Es erstreckt sich eine Längsrichtung X, welche auch als Tiefe X oder als Länge X bezeichnet werden kann. Senkrecht zur Längsrichtung X erstreckt sich eine Querrichtung Y, welche auch als Breite Y bezeichnet werden kann. Senkrecht sowohl zur Längsrichtung X als auch zur Querrichtung Y erstreckt sich eine vertikale Richtung Z, welche auch als Höhe Z bezeichnet werden kann. Die Längsrichtung X und die Querrichtung Y bilden gemeinsam die Horizontale X, Y, welche auch als horizontale Ebene X, Y bezeichnet werden kann.

Eine erfindungsgemäße Matratzenwaschvorrichtung 1 gemäß des ersten Ausführungsbeispiels weist eine Wanneneinheit 10 auf. Die Wanneneinheit 10 weist einen Innenraum 10a auf, welcher in der Längsrichtung X und in der Querrichtung Y jeweils von zwei Seitenwänden 10b und in der vertikalen Richtung Z von unten durch einen Boden 10c eingeschlossen wird. Der somit quaderförmig ausgebildete Innenraum 10a der Wanneneinheit 10 nimmt eine Reinigungsflüssigkeit 2 auf, welche aus Wasser und einem Reinigungsmittel besteht und auch als Waschmittellösung 2 bezeichnet werden kann. Beispielsweise der Boden 10c der Wanneneinheit 10 kann dabei beheizbar ausgebildet sein. Dies kann zum Beispiel elektrisch mittels mehrerer Heizschlangen erfolgen, welche innerhalb des Innenraums 10a am Boden 10c der Wanneneinheit angeordnet sind und in die Reinigungsflüssigkeit 2 hineinragen.

Die Reinigungsflüssigkeit 2 kann in einem Zulaufspeicher 16 als Zulauftank 16 vorbereitet bzw. bevorratet und über einen Zulauf 16a bei Bedarf in den Innenraum 10a der Wanneneinheit 10 geleitet werden. Die Reinigungsflüssigkeit 2 kann ferner über einen Ablauf 17a einem Ablaufspeicher 17 in Form eines Ablauftanks 17 zugeführt werden.

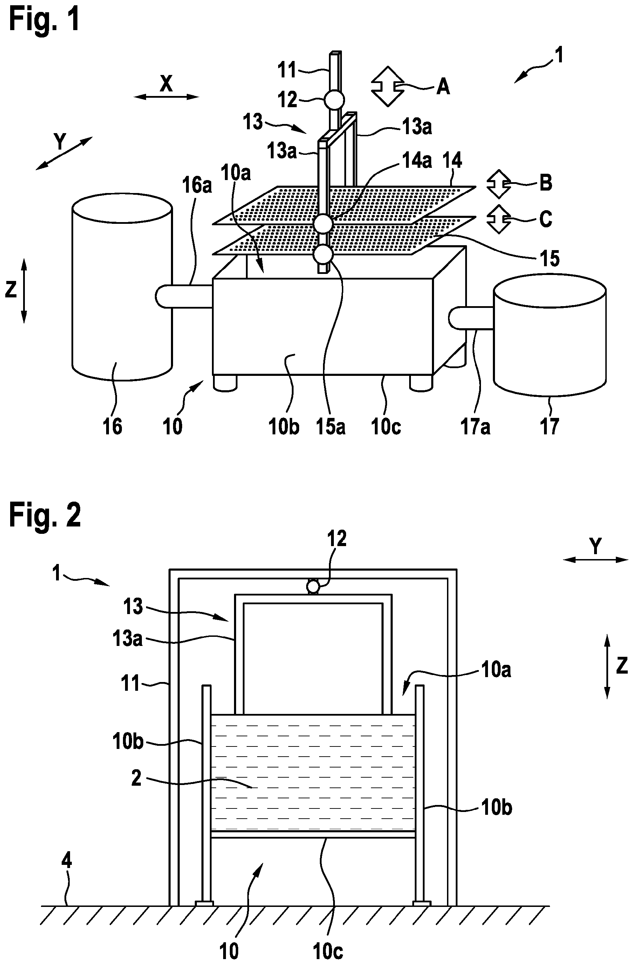

Die Matratzenwaschvorrichtung 1 weist ferner ein Gestell 11 auf, welches die Wanneneinheit 10 in der Längsrichtung X etwa mittig beidseitig bügelförmig bzw. U-förmig umgibt. Das Gestell 11 ist mit seinen beiden offenen Enden in der Querrichtung Y beidseitig der Wanneneinheit 10 feststehend auf einem Untergrund 4 der Wanneneinheit 10 angeordnet. Der mittlere Abschnitt des Gestells 11 überragt in der Querrichtung Y die Wanneneinheit 10 mit einem deutlichen Abstand in der vertikalen Richtung Z, siehe zum Beispiel Figuren 2 bis 5.

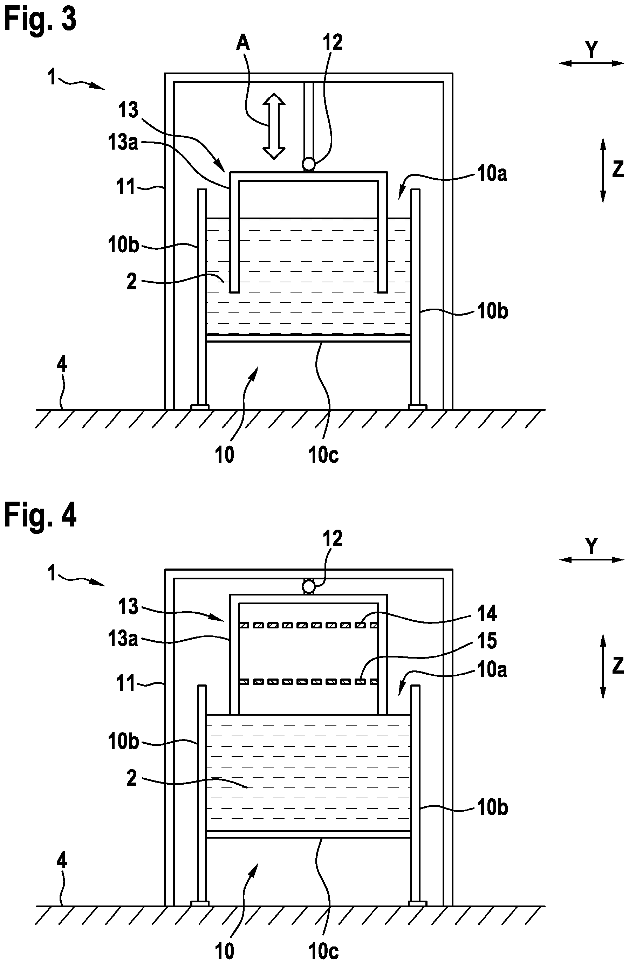

In der vertikalen Richtung Z unterhalb des Gestells 11 ist eine Hubeinheit 13 der Matratzenwaschvorrichtung 1 angeordnet. Ein Rahmen 13a der Hubeinheit 13 ist über einen Hubantrieb 12 mit dem Gestell 11 etwa mittig in der Querrichtung Y derart verbunden, dass die Hubeinheit 13 gegenüber dem Gestell 11 mit einer Hubbewegung A in der vertikalen Richtung Z in den Innenraum 10a der Wanneneinheit 10 hinein sowie in der entgegengesetzten Richtung mit der Hubbewegung A aus dem Innenraum 10a der Wanneneinheit 10 heraus verfahren werden kann, siehe zum Beispiel Figuren 2 und 3. Der Hubantrieb 12 kann beispielsweise mittels eines hydraulischen oder pneumatischen Zylinders umgesetzt werden.

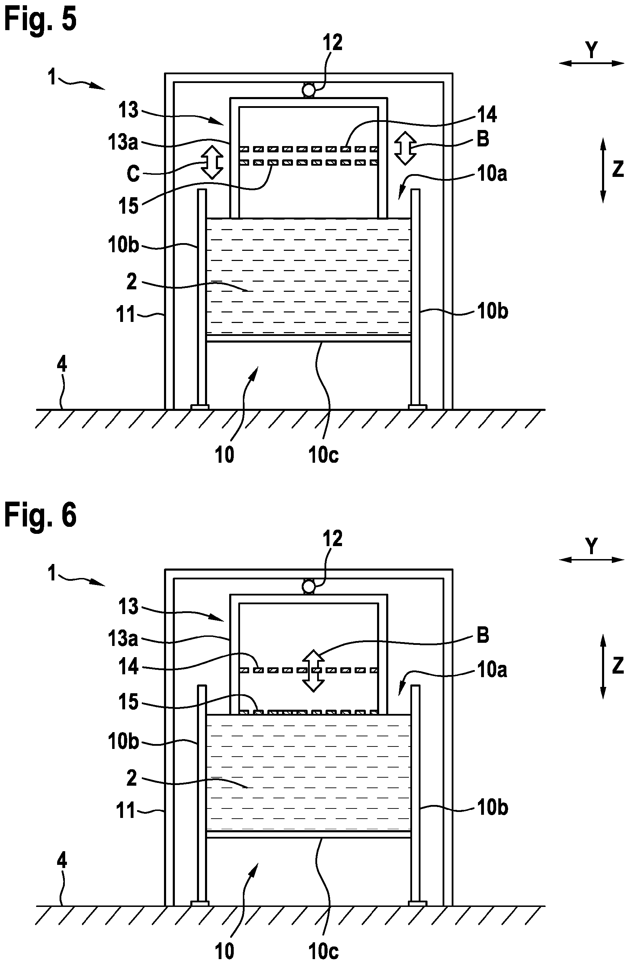

Der Rahmen 13a der Hubeinheit 13 ist ebenfalls bügelförmig bzw. U-förmig und in der vertikalen Richtung Z nach unten hin offen ausgebildet. An den beiden parallel zueinander verlaufenden offenen Enden des Rahmens 13a der Hubeinheit 13 sind jeweils einander gegenüberliegend zwei Paare von Antrieben 14a, 15a angeordnet, welche jeweils zwischen sich in der Querrichtung Y ein Stauchelement 14, 15 aufnehmen. Die beiden Stauchelemente 14, 15 erstrecken sich dabei flächig in der Horizontalen X, Y und können daher auch als Stauchböden 14, 15 bezeichnet werden. Die beiden Stauchelemente 14, 15 sind als Lochbleche 14, 15 ausgebildet. Die beiden Stauchelemente 14, 15 können mit ihren jeweiligen Paaren von Antrieben 14a, 15a unabhängig voneinander in der vertikalen Richtung Z mittels Hubbewegungen B, C an dem Rahmen 13a der Hubeinheit 13 entlang verfahren werden, siehe zum Beispiel Figur 1 und 5.

Zwischen den beiden Stauchelementen 14, 15 kann wenigstens eine Matratze 3 bzw. wenigstens ein Matratzenkern 3 aufgenommen und zusammen mit dem Hubeinheit 13 mittels der Hubbewegung A in der vertikalen Richtung Z in die Reinigungsflüssigkeit 2 im Innenraum 10a der Wanneneinheit 10 verfahren werden. Ebenso kann die Matratze 3 bzw. der Matratzenkern 3 von der Hubeinheit 13 auch wieder aus dem Innenraum 10a der Wanneneinheit 10 heraus verfahren werden. Innerhalb der Hubeinheit 13 kann das erste, obere Stauchelement 14 mittels der Hubbewegung B in der vertikalen Richtung Z nach oben und das zweite, untere Stauchelement 15 mittels der Hubbewegung C in der vertikalen Richtung Z entgegengesetzt nach unten verfahren werden, siehe zum Beispiel Figuren 4 und 5. Hierdurch können die beiden Stauchelemente 14, 15 geöffnet werden, um ein Einlegen oder Entnehmen der Matratze 3 bzw. des Matratzenkerns 3 zu ermöglichen. Auch kann hierdurch ein Entstauchen erfolgen, wie weiter unten näher beschrieben werden wird. Umgekehrt kann das erste, obere Stauchelement 14 mittels der Hubbewegung B in der vertikalen Richtung Z innerhalb der Hubeinheit 13 nach unten und das zweite, untere Stauchelement 15 mittels der Hubbewegung C in der vertikalen Richtung Z entgegengesetzt nach oben verfahren werden, wodurch eine zwischen den beiden Stauchelementen 14, 15 aufgenommene Matratze 3 bzw. Matratzenkern 3 in der vertikalen Richtung Z senkrecht zu ihrer flächigen Erstreckung gestaucht werden kann, siehe zum Beispiel Figur 5, wie ebenfalls weiter unten näher beschrieben werden wird.

Alternativ kann gemäß eines zweiten Ausführungsbeispiels der erfindungsgemäßen Matratzenwaschvorrichtung 1 das zweite Stauchelement 15 feststehend an dem Rahmen 13a der Hubeinheit 13 angeordnet sein, sodass die Hubbewegung B des ersten Stauchelements 14 relativ zu dem feststehenden zweiten Stauchelement 15 erfolgt. Hierdurch kann auf den Antrieb 15a des zweiten Stauchelements 15 verzichtet werden, was die Kosten, das Gewicht, den Bauraum und bzw. oder den Energieverbrauch der Hubeinheit 13 reduzieren kann. Auch kann dies die Ansteuerung des verbleibenden Antriebs 14a des ersten Stauchelements 14 vereinfachen.

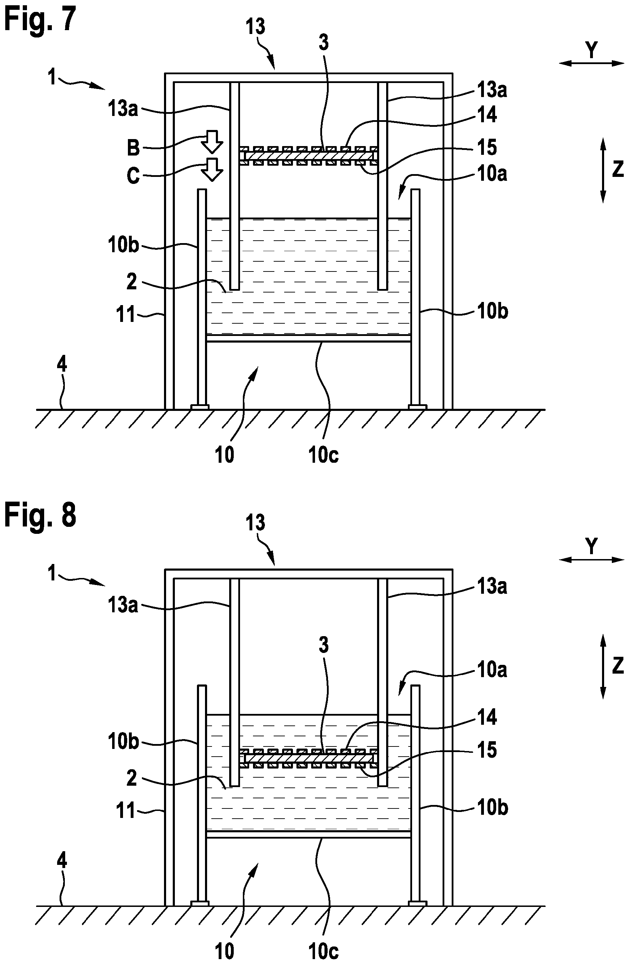

Alternativ kann gemäß eines dritten Ausführungsbeispiels der erfindungsgemäßen Matratzenwaschvorrichtung 1 der Rahmen 13a der Hubeinheit 13 feststehend mit dem Gestell 10 verbunden sein. Die Verfahrwege der beiden Stauchelemente 14, 15 können dann in der vertikalen Richtung Z dann derart länger bemessen sein, dass die beiden Stauchelemente 14, 15 selbst mittels der Hubbewegungen B, C in die Reinigungsflüssigkeit 2 hinein und aus der Reinigungsflüssigkeit 2 heraus verfahren werden können, siehe zum Beispiel Figuren 7 und 8, und dabei die Matratze 3 bzw. den Matratzenkern 3 mit sich mitführen können. Zusätzlich können die beiden Stauchelemente 14, 15 zwischen sich das Stauchen und Entstauchen durchführen, wie zuvor beschrieben. Hierdurch kann auf den Hubantrieb 12 zwischen dem Rahmen 13a der Hubeinheit 13 und dem Gestell 11 verzichtet werden, was die Kosten, das Gewicht, den Bauraum und bzw. oder den Energieverbrauch der Hubeinheit 13 reduzieren kann.

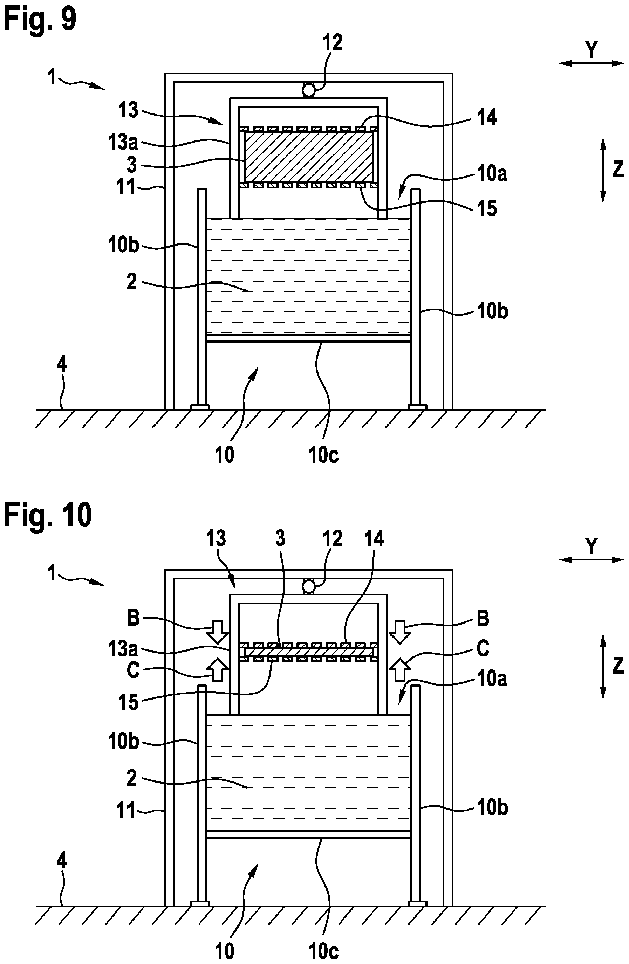

Mittels der erfindungsgemäßen Matratzenwaschvorrichtung 1 gemäß des ersten Ausführungsbeispiels kann ein erfindungsgemäßes Verfahren wie folgt ausgeführt werden:
Außerhalb der Reinigungsflüssigkeit 2, d. h. in der vertikalen Richtung Z oberhalb der Wanneneinheit 10, erfolgt ein Aufnehmen 100 der zu waschenden Matratze 3 bzw. des zu waschenden Matratzenkerns 3 zwischen den beiden Stauchelementen 14, 15 der Hubeinheit 13, welche hierzu in der vertikalen Richtung Z auseinander bzw. von einander weg verfahren sind, siehe zum Beispiel Figur 9. Die Matratze 3 bzw. der Matratzenkern 2 kann hierzu von einer Person zwischen den beiden Stauchelementen 14, 15 angeordnet werden. Hierdurch kann die zu waschende Matratzen 3 bzw. der zu waschende Matratzenkern 3 von der Hubeinheit 13 sich in der Horizontalen X, Y flächig und damit sich flach erstreckend aufgenommen werden.

Die beiden Stauchelemente 14, 15 werden nun mittels der Hubbewegungen B, C aufeinander zu verfahren, wodurch die Matratze 3 bzw. der Matratzenkern 3 in der vertikalen Richtung Z zusammengedrückt, zusammengepresst bzw. komprimiert wird. Hierdurch erfolgt ein erstes Stauchen 100a der Matratze 3 bzw. des Matratzenkerns 3 senkrecht zu ihrer bzw. zu seiner flächigen Erstreckungsrichtung außerhalb bzw. in der vertikalen Richtung Z oberhalb der Reinigungsflüssigkeit 2, siehe Figur 10.

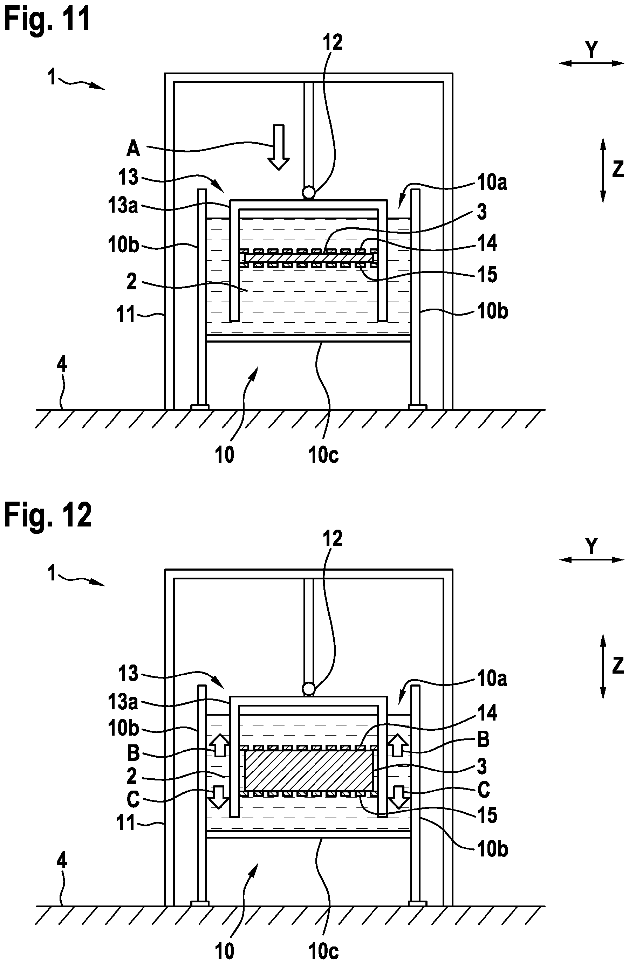

In dieser Konstellation wird nun die Hubeinheit 13 als Ganzes mittels der Hubbewegung A in der vertikalen Richtung Z nach unten in die Reinigungsflüssigkeit 2 hinein verfahren, bis die beiden Stauchelemente 14, 15 vollständig innerhalb der Reinigungsflüssigkeit 2 angeordnet sind. Hierdurch erfolgt ein Eintauchen 200a der Matratze 3 bzw. des Matratzenkerns 3 in die Reinigungsflüssigkeit 2, siehe Figur 11.

Innerhalb der Reinigungsflüssigkeit 2 werden nun die beiden Stauchelemente 14, 15 mittels der Hubbewegungen B, C in der entgegengesetzten Richtung voneinander weg verfahren, wodurch der gestauchte Zustand der Matratze 3 bzw. des Matratzenkerns 3 wieder aufgehoben wird. Hierdurch erfolgt ein erstes Entstauchen 300 der Matratze 3 bzw. des Matratzenkerns 3 innerhalb der Reinigungsflüssigkeit 2, siehe Figur 12, wodurch ein Teil der Reinigungsflüssigkeit 2 in das Material der Matratze 3 bzw. des Matratzenkerns 3 hineingesogen wird. Hierdurch kann die Reinigungsflüssigkeit 2 tief bzw. vollständig in das Material der Matratze 3 bzw. des Matratzenkerns 3 eindringen und so auch angetrocknete Verschmutzungen dort lösen und in sich aufnehmen.

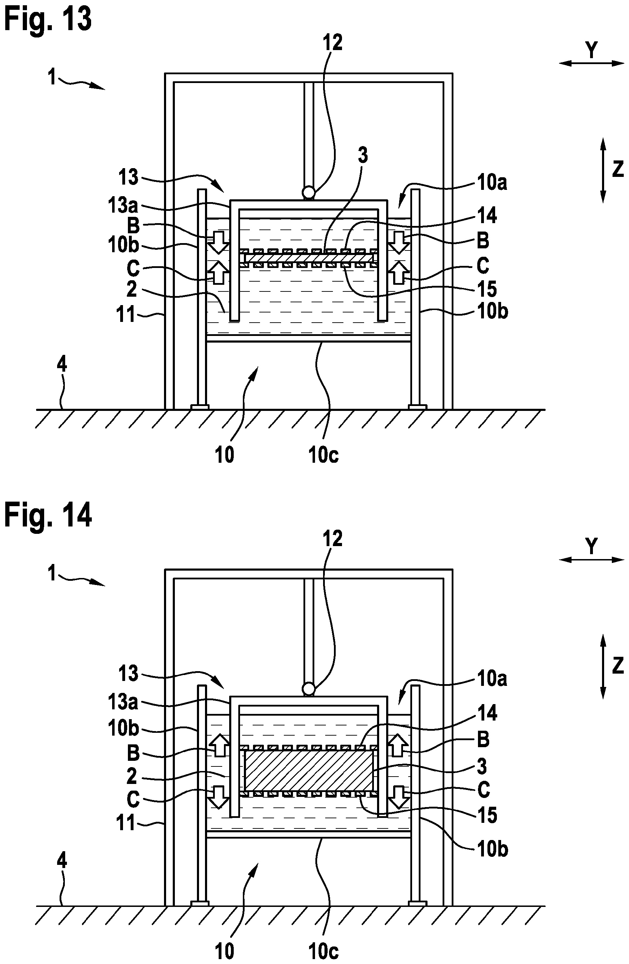

Nun werden die beiden Stauchelemente 14, 15 mittels der Hubbewegungen B, C erneut aufeinander zu bewegt, wodurch die Matratze 3 bzw. der Matratzenkern 3 erneut zusammengedrückt wird. Hierdurch erfolgt ein zweites Stauchen 400a der Matratze 3 bzw. des Matratzenkerns 3 senkrecht zu ihrer bzw. zu seiner flächigen Erstreckungsrichtung innerhalb der Reinigungsflüssigkeit 2, siehe Figur 13, wodurch die Reinigungsflüssigkeit 2 wieder aus dem Material der Matratze 3 bzw. des Matratzenkerns 3 herausgepresst wird. Da die Reinigungsflüssigkeit 2 hierbei die gelösten Verschmutzungen mit sich mitgeführt, kann hierdurch ein tiefwirkender Waschvorgang des Materials der Matratze 3 bzw. des Matratzenkerns 3 durchgeführt werden.

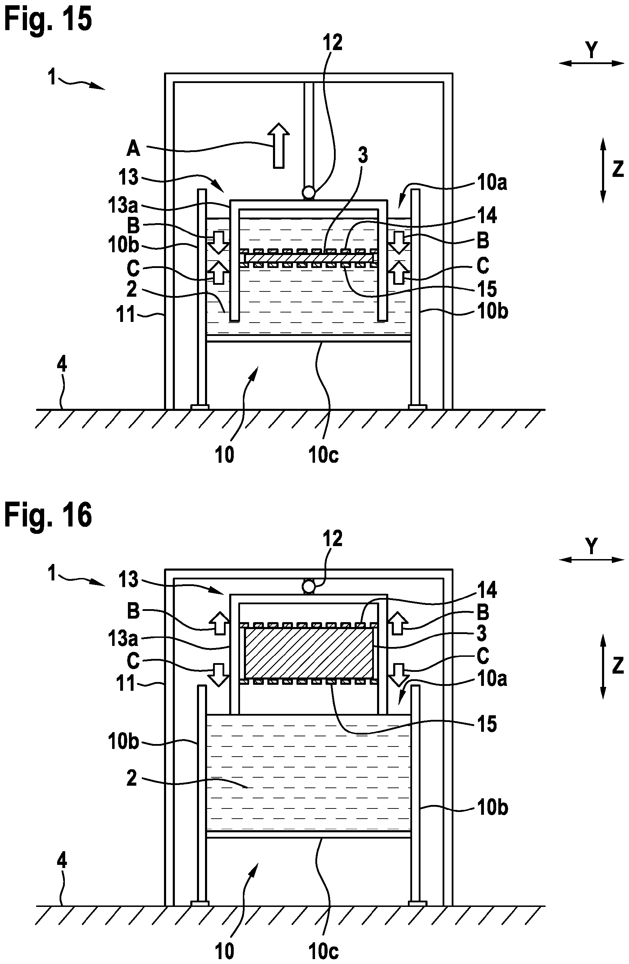

Die Schritte des (ersten) Stauchens 200b und des (ersten) Entstauchens 300 können dabei mehrfach innerhalb der Reinigungsflüssigkeit 2 ausgeführt werden, um die hierdurch bewirkte Reinigungswirkung zu erhöhen, siehe Figuren 14 und 15.

Abschließend werden dabei die beiden Stauchelemente 14, 15 wieder mittels der Hubbewegungen B, C aufeinander zu bewegt und die Matratze 3 bzw. Matratzenkern 3 hierdurch erneut gestaucht. Im gestauchten Zustand wird dann die Matratze 3 bzw. der Matratzenkern 3 von der Hubeinheit 13 mittels der Hubbewegung A in der vertikalen Richtung Z nach oben aus dem Innenraum 10a der Wanneneinheit 10 heraus verfahren, siehe Figur 15. Hierdurch erfolgt ein Herausbewegen 500a der Matratze 3 bzw. des Matratzenkerns 3 aus der Reinigungsflüssigkeit 2.

In der vertikalen Richtung Z oberhalb der Wanneneinheit 10 werden nun die beiden Stauchelemente 14, 15 mittels der Hubbewegungen B, C wieder auseinander bzw. voneinander weg verfahren, siehe Figur 16, wodurch ein zweites bzw. ein weiteres Entstauchen 600 der Matratze 3 bzw. des Matratzenkerns 3 außerhalb der Reinigungsflüssigkeit 2 erfolgt.

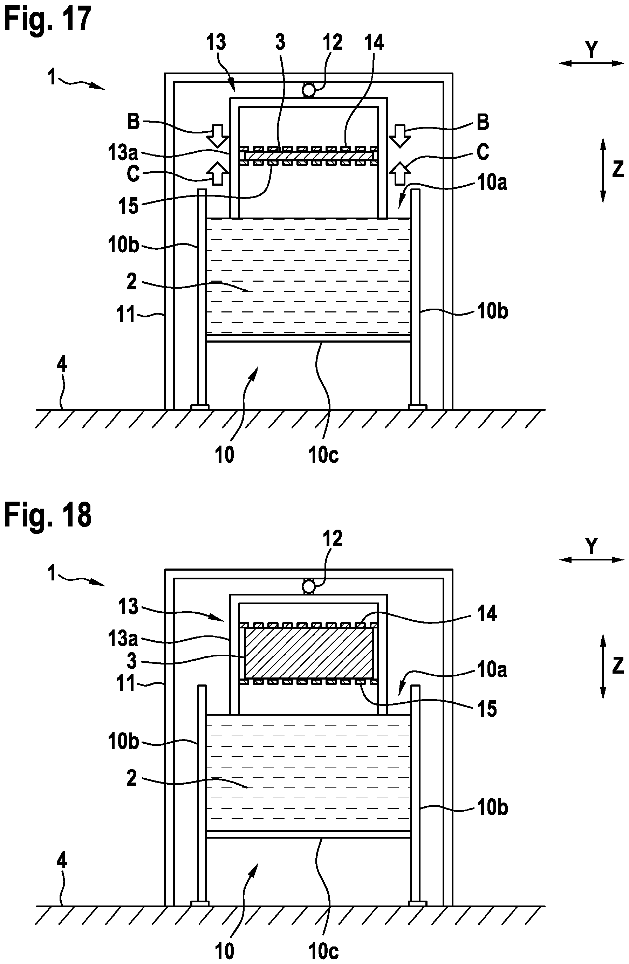

Die Schritte des zweiten bzw. des weiteren Stauchens 500b und des zweiten bzw. weiteren Entstauchens 600 außerhalb der Reinigungsflüssigkeit 2 können dabei mehrfach ausgeführt werden, um weitere Reinigungsflüssigkeit 2 aus dem Material der Matratze 3 bzw. des Matratzenkerns 3 durch Herauspressens zu entfernen, siehe Figuren 17 und 18.

Schließlich werden die beiden Stauchelemente 14, 15 mittels der Hubbewegungen B, C wieder von einander weg bzw. auseinander verfahren und hierdurch die Matratze 3 bzw. Matratzenkern 3 von den beiden Stauchelementen 14, 15 freigegeben. Hierdurch kann außerhalb der Reinigungsflüssigkeit 2 ein Entnehmen 700 der Matratze 3 bzw. des Matratzenkerns 3 aus der Hubeinheit 13 zum Beispiel durch eine Person erfolgen.

Alternativ können das Eintauchen 100b der Matratze 3 bzw. des Matratzenkerns 3 in die Reinigungsflüssigkeit 2 und das erste Stauchen 200b der Matratze 3 bzw. des Matratzenkerns 3 senkrecht zu ihrer bzw. zu seiner flächigen Erstreckungsrichtung auch innerhalb der Reinigungsflüssigkeit 2 erfolgen. Ebenso können alternativ auch das Herausbewegen 400b der Matratze 3 bzw. des Matratzenkerns 3 aus der Reinigungsflüssigkeit 2 und das zweite Stauchen 500b der Matratze 3 bzw. des Matratzenkerns 3 senkrecht zu ihrer bzw. zu seiner flächigen Erstreckungsrichtung außerhalb der Reinigungsflüssigkeit 2 erfolgen.

Während der zuvor beschriebenen Verfahrensschritte kann die Reinigungsflüssigkeit 2 aufgeheizt werden, um die reinigende Wirkung der Reinigungsflüssigkeit 2 zu erhöhen bzw. zu erhalten.

### BEZUGSZEICHENLISTE (Teil der Beschreibung)

- A: Hubbewegung des Hubantriebs 12
- B: Hubbewegung des Antriebs 14a des ersten Presselements 14
- C: Hubbewegung des Antriebs 15a des ersten Presselements 15

- X: Längsrichtung; Tiefe; Länge
- Y: Querrichtung; Breite
- Z: vertikale Richtung; Höhe
- X, Y: Horizontale; horizontale Ebene

- 1: Matratzenwaschvorrichtung; Matratzen-Stauch-Tauch-Waschmaschine
- 10: Wanneneinheit
- 10a: Innenraum der Wanneneinheit 10
- 10b: Seitenwände der Wanneneinheit 10
- 10c: Boden der Wanneneinheit 10
- 11: Gestell
- 12: Hubantrieb
- 13: Hubeinheit
- 13a: Rahmen der Hubeinheit 13
- 14: erstes Stauchelement; erster Stauchboden; erstes Lochblech
- 14a: Antrieb des ersten Stauchelements 14
- 15: zweites Stauchelement; zweiter Stauchboden; zweites Lochblech
- 15a: Antrieb des zweiten Stauchelements 15
- 16: Zulaufspeicher; Zulauftank
- 16a: Zulauf
- 17: Ablaufspeicher; Ablauftank
- 17a: Ablauf

- 2: Reinigungsflüssigkeit; Waschmittellösung

- 3: Matratze; Matratzenkern

- 4: Untergrund

## Patentansprüche

1. Verfahren zum Waschen wenigstens einer Matratze (3) oder wenigstens eines Matratzenkerns (3) mit wenigstens den Schritten:
erstes Stauchen (100a; 200b) der Matratze (3) bzw. des Matratzenkerns (3) zumindest im Wesentlichen senkrecht zu ihrer bzw. zu seiner flächigen Erstreckungsrichtung innerhalb oder außerhalb einer Reinigungsflüssigkeit (2),
erstes Entstauchen (300) der Matratze (3) bzw. des Matratzenkerns (3) innerhalb der Reinigungsflüssigkeit (2) und
zweites Stauchen (400a; 500b) der Matratze (3) bzw. des Matratzenkerns (3) zumindest im Wesentlichen senkrecht zu ihrer bzw. zu seiner flächigen Erstreckungsrichtung innerhalb oder außerhalb der Reinigungsflüssigkeit (2).

2. Verfahren nach Anspruch 1 mit wenigstens den weiteren Schritten:
erstes Stauchen (100a) der Matratze (3) bzw. des Matratzenkerns (3) zumindest im Wesentlichen senkrecht zu ihrer bzw. zu seiner flächigen Erstreckungsrichtung außerhalb der Reinigungsflüssigkeit (2) und
Eintauchen (200a) der Matratze (3) bzw. des Matratzenkerns (3) in die Reinigungsflüssigkeit (2).

3. Verfahren nach Anspruch 1 mit wenigstens den weiteren Schritten:
Eintauchen (100b) der Matratze (3) bzw. des Matratzenkerns (3) in die Reinigungsflüssigkeit (2) und
erstes Stauchen (200b) der Matratze (3) bzw. des Matratzenkerns (3) zumindest im Wesentlichen senkrecht zu ihrer bzw. zu seiner flächigen Erstreckungsrichtung innerhalb der Reinigungsflüssigkeit (2).

4. Verfahren nach einem der Ansprüche 1 bis 3 mit wenigstens den weiteren Schritten:
zweites Stauchen (400a) der Matratze (3) bzw. des Matratzenkerns (3) zumindest im Wesentlichen senkrecht zu ihrer bzw. zu seiner flächigen Erstreckungsrichtung innerhalb der Reinigungsflüssigkeit (2) und
Herausbewegen (500a) der Matratze (3) bzw. des Matratzenkerns (3) aus der Reinigungsflüssigkeit (2).

5. Verfahren nach einem der Ansprüche 1 bis 3 mit wenigstens den weiteren Schritten:
Herausbewegen (400b) der Matratze (3) bzw. des Matratzenkerns (3) aus der Reinigungsflüssigkeit (2) und
zweites Stauchen (500b) der Matratze (3) bzw. des Matratzenkerns (3) zumindest im Wesentlichen senkrecht zu ihrer bzw. zu seiner flächigen Erstreckungsrichtung außerhalb der Reinigungsflüssigkeit (2).

6. Verfahren nach einem der vorangehenden Ansprüche mit wenigstens dem weiteren Schritt:
zweites Entstauchen (600) der Matratze (3) bzw. des Matratzenkerns (3) außerhalb der Reinigungsflüssigkeit (2).

7. Verfahren nach einem der vorangehenden Ansprüche mit wenigstens den weiteren Schritten:
außerhalb der Reinigungsflüssigkeit (2), Aufnehmen (100) der Matratze (3) bzw. des Matratzenkerns (3), vorzugsweise in einer Hubeinheit (13), und/oder
außerhalb der Reinigungsflüssigkeit (2), Entnehmen (700) der Matratze (3) bzw. des Matratzenkerns (3), vorzugsweise aus der Hubeinheit (13).

8. Verfahren nach einem der vorangehenden Ansprüche, **dadurch gekennzeichnet, dass**
die Schritte des Stauchens (200b) und des Entstauchens (300) mehrfach innerhalb der Reinigungsflüssigkeit (2) ausgeführt werden.

9. Verfahren nach einem der vorangehenden Ansprüche, **dadurch gekennzeichnet, dass**
die Schritte des zweiten Stauchens (500b) und des zweiten Entstauchens (600) außerhalb der Reinigungsflüssigkeit (2) mehrfach ausgeführt werden.

10. Verfahren nach einem der Ansprüche 2 bis 9, **dadurch gekennzeichnet, dass**
das Eintauchen (100b; 200a) in die Reinigungsflüssigkeit (2) zumindest im Wesentlichen senkrecht zur flächigen Erstreckungsrichtung der Matratze (3) bzw. des Matratzenkerns (3), vorzugsweise in der vertikalen Richtung (Z), erfolgt und/oder
das Herausbewegen (400b; 500a) aus der Reinigungsflüssigkeit (2) zumindest im Wesentlichen senkrecht zur flächigen Erstreckungsrichtung der Matratze (3) bzw. des Matratzenkerns (3), vorzugsweise in der vertikalen Richtung (Z), erfolgt.

11. Verfahren nach einem der vorangehenden Ansprüche, **dadurch gekennzeichnet, dass** die Reinigungsflüssigkeit (2) aufgeheizt ist.

12. Matratzenwaschvorrichtung (1), vorzugsweise zur Durchführung eines Verfahrens nach einem der vorangehenden Ansprüche,
mit wenigstens einer Wanneneinheit (10), welche ausgebildet ist, eine Reinigungsflüssigkeit (2) in einem Innenraum (10a) aufzunehmen, und
mit wenigstens einer Hubeinheit (13), welche ausgebildet ist, wenigstens eine Matratze (3) oder wenigstens einen Matratzenkern (3) aufzunehmen,
wobei die Hubeinheit (13) ferner ausgebildet ist, die aufgenommene Matratze (3) bzw. den aufgenommenen Matratzenkern (3), vorzugsweise zumindest im Wesentlichen senkrecht zu ihrer bzw. zu seiner flächigen Erstreckungsrichtung, besonderes vorzugsweise in der vertikalen Richtung (Z), vorzugsweise gegenüber einem Gestell (11), in den Innenraum (10a) der Wanneneinheit (10) hinein und/oder aus dem Innenraum (10a) der Wanneneinheit (10) heraus zu bewegen,
wobei die Hubeinheit (13) ferner ausgebildet ist, die aufgenommene Matratze (3) bzw. den aufgenommenen Matratzenkern (3) zumindest im Wesentlichen senkrecht zu ihrer bzw. zu seiner flächigen Erstreckungsrichtung, vorzugsweise in der vertikalen Richtung (Z), zu stauchen und zu entstauchen.

13. Matratzenwaschvorrichtung (1) nach Anspruch 12, **dadurch gekennzeichnet, dass**
die Hubeinheit (13) wenigstens ein erstes Stauchelement (14) und ein zweites Stauchelement (15) aufweist, welche ausgebildet sind, zwischen sich die Matratze (3) bzw. den Matratzenkern (3), vorzugsweise zumindest im Wesentlichen senkrecht zu ihrer bzw. zu seiner flächigen Erstreckungsrichtung, besonderes vorzugsweise in der vertikalen Richtung (Z), aufzunehmen,
wobei wenigstens das erste Stauchelement (14) ausgebildet ist, innerhalb der Hubeinheit (13) relativ zum zweiten Stauchelement (15) zumindest im Wesentlichen senkrecht zur flächigen Erstreckungsrichtung der aufgenommenen Matratze (3) bzw. des aufgenommenen Matratzenkerns (3), vorzugsweise in der vertikalen Richtung (Z), bewegt zu werden und hierdurch die aufgenommene Matratze (3) bzw. den aufgenommenen Matratzenkern (3) zumindest im Wesentlichen senkrecht zu ihrer bzw. zu seiner flächigen Erstreckungsrichtung, vorzugsweise in der vertikalen Richtung (Z), zu stauchen und zu entstauchen.

14. Matratzenwaschvorrichtung (1) nach Anspruch 13, **dadurch gekennzeichnet, dass**
das zweite Stauchelement (15) feststehend an der Hubeinheit (13) angeordnet ist oder
das zweite Stauchelement (15) ausgebildet ist, innerhalb der Hubeinheit (13) relativ zum ersten Stauchelement (14) zumindest im Wesentlichen senkrecht zur flächigen Erstreckungsrichtung der aufgenommenen Matratze (3) bzw. des aufgenommenen Matratzenkerns (3), vorzugsweise in der vertikalen Richtung (Z), bewegt zu werden und hierdurch die aufgenommene Matratze (3) bzw. den aufgenommenen Matratzenkern (3) zumindest im Wesentlichen senkrecht zu ihrer bzw. zu seiner flächigen Erstreckungsrichtung, vorzugsweise in der vertikalen Richtung (Z), zu stauchen und zu entstauchen.

15. Matratzenwaschvorrichtung (1) nach einem der Ansprüche 12 bis 14, **dadurch gekennzeichnet, dass**
die Wanneneinheit (10), vorzugweise wenigstens eine Seitenwand (10b) und/oder ein Boden (10c) der Wanneneinheit (10), zumindest abschnittsweise beheizbar ausgebildet ist.
